# EUROPEAN PATENT APPLICATION

(11) **EP 4 643 831 A1**
(43) Date of publication of application: **05.11.2025**
(21) Application number: 23910341.9
(22) Date of filing: 21.12.2023
(51) Int. Cl.: A61F 9/00

(54) **SYRINGE SLEEVE, SLEEVE WITH NEEDLE, INJECTION ASSEMBLY, AND USE METHOD THEREFOR**

(30) Priority: 30.12.2022 CN 202211738981
(71) Applicant: Chengdu Origen Biotechnology Co., Ltd., Chengdu, Sichuan 610037 (CN)
(72) Inventor: KE, Xiao, Chengdu, Sichuan 610037 (CN); ZHENG, Qiang, Chengdu, Sichuan 610037 (CN); QIN, Yingfei, Chengdu, Sichuan 610037 (CN)
(74) Representative: reuteler & cie SA
(86) International application number: PCT/CN2023/140680
(87) International publication number: WO 2024/140415

(57) **Abstract**

A syringe sleeve, comprising a positioning cylinder (2) for being fixed to a syringe cylinder flange (41), and a connecting cylinder (1) comprising a connecting end (12) and an injection end (11). The connecting end (12) is used for being connected to the positioning cylinder (2), and the injection end (11) is used for limiting the part of the tip of a needle (3) extending out of the connecting cylinder (1). Further provided are a sleeve with a needle and an injection assembly. The syringe sleeve, the sleeve with a needle, and the injection assembly provided can conveniently limit the part of the tip of the needle (3) extending out of the connecting cylinder (1), thereby quickly and accurately adjusting a syringe (4) and preventing the syringe (4) from moving back and being deviated from the injection position.

## Description

### Field

The present disclosure relates to the technical field of ophthalmic medical instruments, in particular to a syringe sleeve, a sleeve with a needle, an injection assembly and a use method therefor.

### Background

An eye is a highly complex visual organ, including an eyeball, a visual pathway, and adnexa, in which the eyeball and the visual pathway complete a visual function. The eyeball is composed of an eyeball wall and eyeball contents, is approximately spherical and has a very complex structure. The eyeball wall is composed of three layers, i.e., an outer layer referred to as fibrous tunic, a middle layer referred to as uvea, and an inner layer referred to as retina. The fibrous tunic is mainly formed by fibrous tissues, and is an outer membrane of the eyeball. The fibrous tunic comprises cornea and sclera, wherein the sclera accounts for 5/6. The sclera is strong but pliable, and formed by dense and interlaced fibers. The thickness of sclera is different at different parts and has individual specificity. In different developmental stages, including child, adult and elderly, the arrangement of scleral collagen fiber bundles of sclera is different in different parts of the eyeball. As an exposed organ, the eye is vulnerable to conjunctival or corneal damages caused by various damaging pathogens. For example, the conjunctiva is a thin and transparent mucosa that has abundant blood vessels, and it covers the inner surface of the eyelid and the front of the eyeball, and is helpful for protecting the eyeball against damages foreign matters and infections. However, the conjunctiva itself is very sensitive, easily stimulated by chemical substances or allergic substances, or infected by viruses and bacteria, thus causing conjunctivitis; in addition, the abundant blood vessels in the conjunctiva can introduce a large quantity of medicaments (> 60%) into the systemic circulation, thereby may easily result in unnecessary tissue toxicity, etc.

At present, fundus oculi diseases are one of the main causes for irreparable visual impairments or injuries, mainly including neovascular and age-related macular degeneration, diabetic retinopathy, diabetic macular edema, central retinal vein occlusion and branch retinal vein occlusion, etc. In the treatment of ocular diseases, in view that tissue barriers (e.g., corneal, conjunctival, blood-aqueous barrier and blood-retinal barrier) limit the delivery of medicaments to the fundus, ophthalmic medicament delivery devices play a key role in medicament delivery. Though existing delivery methods, such as ocular surface administration and intravitreal injection (IVT), are convenient, it is difficult to use such methods to deliver medicaments to fundus lesions efficiently and safely. In addition, if puncturing is performed in eye injection at an incorrect position or angle, it may lead to complications, such as intraocular hemorrhage or retinal injury, which may lead to cataracts and retinal detachment. If medicament leakage happens, it may cause infection of other tissues in eyes.

As described above, it has always been a difficult problem to deliver medicaments to eyes owing to the particularities of eye structure. It is especially difficult to deliver medicaments to the suprachoroidal space, owing to the structure of the suprachoroidal space. The suprachoroidal space is a potential cavity gap between the sclera and the choroid, and there is no obvious cavity gap when there is no fluid and/or tissue separation. When a fluid or other material is accumulated between the choroid and the sclera, the suprachoroidal space may appear in that area. Therefore, fluid accumulation is intentionally created by delivering, injecting and/or infusing a medicament formulation into the suprachoroidal space to further create and/or enlarge the suprachoroidal space formed by the separation of the choroid from the sclera. Local choroidal hemorrhage may occur in injection during medicament administration to the suprachoroidal space, resulting in retinal damages; in addition, the injection may cause complications, such as endophthalmitis, scleral dilatation, wound abscess, etc., and may cause high intraocular pressure and cataract occasionally.

Injection into ocular tissues has high requirements for the accuracy of the distance between the syringe itself and the ocular tissues, and the penetration depth and position of the needle, etc. The operator may achieve accurate positioning before the injection. However, in existing methods of injection into ocular tissues, the penetration depth and position of the needle may be confirmed and measured repeatedly before the injection. In order to meet the requirements for accuracy when a syringe is used, it often takes a lot of time and labor for the syringe operator to complete the positioning of the syringe in the eye since it is inconvenient to make adjustments; on the other hand, to enable the needle to pierce into the eye, the operator may exert certain force on the syringe and wear gloves during the operation, but slippage may occur easily between the syringe and the gloves. In addition, when the needle pierces into the eye tissues, it is difficult to position the syringe accurately, and the needle may move backward or displace from the injection position under the effect of the eye tissues; consequently, the desired injection effect can't be achieved, or even accidents may occur.

### Summary

An object of the embodiments of the present application is to provide a syringe sleeve, a sleeve with a needle and an injection assembly, so as to overcome the defect that the precision of injection can't be adjusted conveniently in existing syringes and realize convenient adjustment of the precision of injection. Another object of the embodiments of the present application is to provide a syringe sleeve, a sleeve with a needle and an injection assembly, so that the injection assembly can be adjusted quickly, accurately, and easily, and it is unnecessary to repeatedly measure and confirm the penetration depth and position of the needle into the eye tissues during use, thereby time and labor can be saved greatly. Yet another object of the embodiments of the present application is to provide a syringe sleeve, a sleeve with a needle and an injection assembly, so that a stable syringe position can be provided by rapid positioning of the injection assembly during the injection into eye tissues, and avoiding needle retraction or injection position deviation.

To attain the above objects, the following technical schemes are provided in the embodiments of the present application:
In a first aspect, a syringe sleeve to be sleeved outside a syringe is provided. The syringe comprises a barrel with a barrel flange and can carry a needle; the syringe sleeve comprises a locating cylinder for being fixedly coupled to the barrel flange and a connecting cylinder, and the connecting cylinder comprises a connecting end for connecting with the locating cylinder and an injection end for limiting a part of the tip of the needle extending out of the connecting cylinder.

With the syringe sleeve, the barrel flange can be fixedly coupled to the locating cylinder by means of the structure of an existing syringe, and then the syringe and the locating cylinder can be coupled, so that the operator's grip operation on the syringe can be changed into the operator's grip operation on the syringe sleeve, thereby the use and operation are more convenient. By positioning the syringe sleeve, the syringe can be located during injection into the eye tissues; thus, a stable syringe position is provided, and backward movement or deviation of the syringe from the injection position is avoided.

In a preferred embodiment of the present application, when the connecting cylinder and the locating cylinder are coupled, the total length of the connecting cylinder and the locating cylinder can be changed by means of the coupling.

The total length of the connecting cylinder and the locating cylinder can be adjusted conveniently by means of the coupling between the connecting cylinder and the locating cylinder. From the initial position and state of the coupling to the final position and state of the coupling, the total length of the connecting cylinder and the locating cylinder can be changed for at least one time, thus, the change of the total length of the connecting cylinder and the locating cylinder is realized. The injection end of the connecting cylinder limits the relative position of the needle, and the tip of the needle can be extending out of the connecting cylinder. The exposed length of the tip of the needle can be adjusted by adjusting the relative positions of the connecting cylinder and the locating cylinder. Since the total length of the connecting cylinder and the locating cylinder in the final coupled state is determined in prefabrication, and the length of the syringe and the length of the needle are also determined in prefabrication, the exposed length of the tip of the needle can be determined by means of parameters in prefabrication. When the connecting cylinder and the locating cylinder are coupled, to the final state, the exposed length of the tip of the needle is the length of the tip of the needle required for injection into the eye tissues, thus, the positioning of the tip of the needle is realized, so that the requirements for injection into the eye tissues are met. Moreover, the connection and adjustment of the syringe sleeve can be completed quickly, and it is unnecessary to make measurements and confirmations repeatedly during use, thus, time and labor are saved greatly.

The term "limiting" herein refers to fixing the part of the tip of the needle exposed from the injection end of the connecting cylinder so as to avoid any change of the exposed length of the tip of the needle or any wobble of the needle in the injection process.

In a preferred embodiment of the present application, the needle has a hub, and an annular boss is provided on the inner side of the injection end of the connecting cylinder, for clamping the hub to limit the needle. By means of the annular boss, the injection end of the connecting cylinder can limit the length of the needle extending out of the connecting cylinder, thereby limit the exposed length of the tip of the needle to meet the injection requirements. With the cooperation of the connecting cylinder and the locating cylinder, the annular boss enables the syringe in the locating cylinder to be quickly coupled with the hub of the needle to form an integral structure and to lock the hub; thus, the backward movement of the needle toward the syringe or deviation of the needle from the injection position under the effect of the tissues at the site of injection, which may result in inaccurate dosage of administration and affect the injection effect, can be avoided in the process of injection into the eye; moreover, the tip of the needle can't move with the push rod of the syringe when the operator exerts push force on the push rod to move the push rod in the syringe because that the hub is clamped by the annular boss, and the needle is prevented from extending excessively in the target tissue in the eye, which may cause injuries of the target tissue in the eye or penetrate through the target tissue in the eye.

In a preferred embodiment of the present application, raised ribs are provided on the periphery of a top end of the hub of the needle for being against the annular boss and limiting the length of the needle extending out of the injection end via the annular boss. By means of the arrangement of the raised ribs that can be against the annular boss, it is convenient to clamp the hub in the injection end of the connecting cylinder.

In a preferred embodiment of the present application, the tip of the injection end is provided with a clamping opening for pressing the eye tissues at the site of injection, and the clamping opening is provided with an annular end face, which has a minimum inner diameter of 1 mm to 3 mm. During use, the needle penetrates into the injection point for injection; the minimum inner diameter of the annular end face of the clamping opening refers to the length of the shortest line segment that passes through the injection point and has two ends located inside the clamping opening respectively, and the injection point is located at the center of the line segment. Experimental results show that when the minimum inner diameter is 1 mm to 3 mm, the area of the eye tissues in the clamping opening is appropriate, and the eye tissues in the clamping opening can form an obvious protrusion, which can ease the injection operation. The clamping opening can facilitate the contact with the eye tissues and fix the position of the syringe, so that the success ratio of injection can be improved and the medicament solution can be delivered to the target site successfully.

In a preferred embodiment of the present application, the injection end of the connecting cylinder has a cavity, one end of the cavity is sealed by the needle seat of the needle, and the other end of the cavity is sealed under a squeezing action between the eye tissues and the clamping opening. With the arrangement of the cavity, when a reflux occurs, the medicament solution in the reflux will be contained in the sealed cavity; when the clamping opening is separated from the eye tissues, the refluxed medicament solution will not leak out of the cavity easily, thus, effectively avoiding the diffusion of the refluxed medication on the ocular surface as a result of injection failure..

In a preferred embodiment of the present application, the side wall of the injection end of the connecting cylinder is provided with a viewing window made of a transparent material, or the injection end is component made of a transparent material; with the arrangement of the viewing window or the component made of a transparent material, it is convenient to observe the reflux of the medicament solution and quickly judge whether the injection is successful or not.

In a preferred embodiment of the present application, the connecting end of the connecting cylinder may be coupled with the locating cylinder through a threaded connection or snap-fitted connection. By means of a threaded connection or snap-fitted connection, the coupling between the connecting cylinder and the locating cylinder can be realized; moreover, the total length can be adjusted for at least one time or can be adjusted continuously from the initial state or incomplete connection state to the final connection state of the connecting cylinder and the locating cylinder, and the total length of the connecting cylinder and the locating cylinder can be adjusted to an expected value, so that the exposed length of the tip of the needle can be tuned according to the sclera thicknesses of different patients to meet the injection requirements.

In a preferred embodiment of the present application, the connecting end of the connecting cylinder is coupled with an end of the locating cylinder facing the needle, or the connecting cylinder is inserted into and coupled with the locating cylinder from an end of the locating cylinder facing the tail end of the syringe push rod. With the two connection methods, different connection modes or different processes of use of the connecting cylinder and the locating cylinder can be provided, and the scope of application of the syringe sleeve is expanded.

In a preferred embodiment of the present application, the connecting end of the connecting cylinder is provided with external threads, the locating cylinder is provided with internal threads, and the connecting end can be coupled with the locating cylinder in a rotating way and adjust the total length of the connecting cylinder and the locating cylinder in a rotating manner. By means of a threaded connection, the relative lengths of the connecting cylinder and the locating cylinder can be adjusted, thus, the total length can be adjusted.

In a preferred embodiment of the present application, in the case that the connecting cylinder is configured to be inserted into and coupled with the locating cylinder from an end of the locating cylinder facing the tail end of the syringe push rod, after the syringe is mounted, the barrel flange abuts against the connecting end of the connecting cylinder and limits the backward movement of the connecting end in the locating cylinder. By clamping the barrel flange to the locating cylinder, the syringe can be integrated with the locating cylinder, and the position of the connecting end of the connecting cylinder in the locating cylinder can be adjusted. The final position of backward movement of the connecting end is the position where the tip of the connecting end abuts against the barrel flange and the connecting end can't move backward further, thus, a function of preventing backward movement of the syringe sleeve is realized.

In a preferred embodiment of the present application, the inner wall of the connecting cylinder is provided with a positioning groove in the axial direction for mutual clamping with an outer side of the barrel or an outer side of the hub of the needle, so that the syringe can move in the axial direction of the connecting cylinder under the guidance of the positioning groove. By means of the positioning groove, the connecting cylinder can provide at least one passage in the axial direction, which can accommodate the outer side of the barrel or the outer side of the hub of the needle, so that the syringe can be guided to move in the connecting cylinder only in the direction of the central axis, and the needle is prevented from contacting with the inner wall of the connecting cylinder during the movement, which is beneficial for keeping the needle clean.

In a preferred embodiment of the present application, a protrusion is provided on the outer side of the hub of the needle, and the shape and size of the protrusion match the cross section of the positioning groove; by means of the fitting between the protrusion structure on the outer side of the hub and the positioning groove, the needle can move axially in the connecting cylinder along with the positioning groove, and the syringe is always kept moving in one direction without wobble under the guidance of the needle when the syringe is placed to the site of injection; thus, the contact between the needle and the inner wall of the connecting cylinder is avoided in the syringe placement process.

In a preferred embodiment of the present application, the locating cylinder is provided with a mounting groove for mounting the barrel flange, so that the barrel flange is clamped into the mounting groove in a rotating manner. The barrel flange can be clamped into the locating cylinder conveniently for connection by means of the mounting groove, so that the barrel and the locating cylinder are firmly connected.

In a preferred embodiment of the present application, the locating cylinder is provided with a flange mounting platform for supporting one side of the barrel flange and a locking lug for clamping the other side of the barrel flange, and a gap between the locking lug and the flange mounting platform forms the mounting groove; with the arrangement of the flange mounting platform and the locking lug, a space for clamping the barrel flange is provided on the locating cylinder, thereby the syringe can be fixed to the locating cylinder conveniently.

In a preferred embodiment of the present application, an arc-shaped protrusion structure is provided at the top of the flange mounting platform or the bottom of the locking lug for locking to one side of the barrel flange; with the arrangement of the arc-shaped protrusion structure, when the barrel flange is rotationally clamped into the mounting groove, the flange mounting platform or the locking lug can be tightly pressed against the surface of the barrel flange, so that the barrel is clamped and locked in the mounting groove, and is kept in the locked state once it is clamped into the mounting groove; thus, the syringe can be prevented from disengaging from the locating cylinder.

In a preferred embodiment of the present application, a limiting structure for fixing the fingers of an operator is provided on the outer side of the locating cylinder, and the limiting structure is a protrusion, notch or ring structure. The outer diameter of an ophthalmic syringe is usually several millimeters, the operator has to wear gloves and manipulate the syringe directly, but it is inconvenient to manipulate the barrel when wearing rubber gloves, often leading to unstable gripping and slippage. With the limiting structure, it is convenient to position the fingers, and the operator can hold the syringe sleeve conveniently to facilitate the operation.

In a preferred embodiment of the present application, the needle is available in a variety of specifications, and the needle shafts of needles of different specifications are different in length, and may be in length of 5 - 10 mm; needles of different specifications are provided by configuring the length of the needle shaft. Thus, the operator can select needles of different specifications according to different situations and needs of different patients.

In a preferred embodiment of the present application, the injection end of the connecting cylinder is provided with a contraction section for clamping the hub of the needle and limiting the needle. The inner opening of the contraction section is small, and the injection end can clamp the top end of the hub to limit the hub from moving further, thus realizing the function of limiting the needle. Besides, the injection end allows the needle shaft of the needle to pass there-through, so that the tip of the needle can be exposed at the injection end of the connecting cylinder after the connecting cylinder is coupling with the locating cylinder. The syringe sleeve can change the original grip operation of the operator on the syringe into a grip operation of the operator on the syringe sleeve, thereby the use and operation are more convenient. The total length of the connecting cylinder and the locating cylinder can be adjusted conveniently by means of the connection between the connecting cylinder and the locating cylinder, thus, the exposed length of the tip of the needle can be adjusted by adjusting the relative positions of the connecting cylinder and the locating cylinder, so that the tip of the needle is positioned and the exposed length of the tip of the needle can meet the requirements of injection into eye tissues.

When the connecting cylinder and the locating cylinder cooperate to adjust the exposed length of the tip of the needle and limit the position of the needle, the syringe in the locating cylinder can be quickly coupling with the hub of the needle to form an integral structure and locks the hub of the needle; thus, the backward movement of the needle toward the syringe or deviation of the needle from the injection position under the effect of the tissues at the site of injection, which may result in inaccurate dosage of administration and affect the injection effect, can be avoided in the process of injection into the eye; moreover, the tip of the needle can't move with the push rod of the syringe when the operator exerts push force on the push rod to move the push rod in the syringe because that the syringe locks the hub, and the needle is prevented from extending excessively in the target tissue in the eye, which may cause injuries of the target tissue in the eye or penetration through the target tissue in the eye.

In a preferred embodiment of the present application, the syringe sleeve couples the locating cylinder and the connecting cylinder, so that the syringe clamps the needle, thereby the injection will not be affected by inaccurate dosage caused by backward movement of the needle toward the syringe or deviation of the needle from the injection position under the effect of the tissues at the injection site in the injection process.

In a preferred embodiment of the present application, the syringe sleeve couples the locating cylinder and the connecting cylinder, so that the tip of the needle doesn't move with the movement of the push rod, and further extension of the needle in the target eye tissue is prevented, thereby injuries of the target eye tissue or penetration through the target eye tissue are prevented.

In a second aspect, a sleeve with a needle is provided. The sleeve is to be sleeved outside a syringe. The syringe comprises a barrel with a barrel flange, and the needle has a hub; the syringe sleeve comprises a locating cylinder for being fixed to the barrel flange and a connecting cylinder, and the connecting cylinder comprises a connecting end for coupling with the locating cylinder and an injection end connected therein with the hub for limiting a part of the tip of the needle extending out of the connecting cylinder.

In a preferred embodiment of the present application, when the injection end and the hub are coupled, the total length of the injection end and the hub can be changed by means of the connection.

The syringe sleeve directly connects the needle with the connecting cylinder, and limits the exposed length of the tip of the needle by means of the injection end. Thus, since the length of the injection end of the connecting cylinder and the length of the needle are determined in prefabrication, the exposed length of the tip of the needle when the connecting cylinder and the needle are in the final state of connection can be determined by parameters in prefabrication. When the connecting cylinder and the needle are connected in the final state, the exposed length of the tip of the needle is the length of the tip of the needle required for eye injection, thus, the tip of the needle can be positioned, thereby the requirements of injection into eye tissues can be met; the connection and adjustment of the sleeve with the needle can be completed quickly, and it is unnecessary to carry out measurement and confirmation repeatedly during use, thus, time and labor are saved greatly.

When the connecting cylinder and the locating cylinder cooperate to adjust the exposed length of the tip of the needle and limit the position of the needle, the syringe in the locating cylinder can be quickly coupled with the hub of the needle to form an integral structure and locks the hub of the needle; thus, the backward movement of the needle toward the syringe or deviation of the needle from the injection position under the effect of the tissues at the site of injection, which may result in inaccurate dosage of administration and affect the injection effect, can be avoided in the process of injection into the eye; moreover, the tip of the needle can't move with the push rod of the syringe when the operator exerts push force on the push rod to move the push rod in the syringe because that the syringe locks the hub, and the needle is prevented from extending excessively in the target tissue in the eye, which may cause injuries of the target tissue in the eye or penetration through the target tissue in the eye.

In a third aspect, an injection assembly is provided. The injection assembly comprises the aforesaid syringe sleeve or the aforesaid sleeve with a needle and a syringe, wherein the syringe is arranged in a connecting cylinder and a locating cylinder, and a barrel flange of the syringe is clamped in an mounting groove; and the length of the needle of the syringe extending out of the connecting cylinder can be adjusted by means of the couple between the connecting cylinder and the locating cylinder or the couple between the connecting cylinder and the hub of the needle. The injection assembly can be coupled with an existing syringe, thereby the length of the tip of the needle for injection into eye tissues can be adjusted conveniently to required length and accuracy to facilitate the operator to operate with one hand, and the use and operation are more convenient; moreover, when the connecting cylinder and the locating cylinder cooperate to adjust and limit the exposed length of the tip of the needle, the syringe in the locating cylinder can be quickly coupled with the hub of the needle to form an integral structure, and the hub of the needle is locked.

In a fourth aspect, a method for using a syringe sleeve is provided. In the method, the aforesaid syringe sleeve or the aforesaid sleeve with a needle is used, and the method comprises the following steps: firstly, connecting the connecting cylinder with the locating cylinder, next, placing the barrel into the connecting cylinder and the locating cylinder, and then clamping the barrel flange into the mounting groove for fixing; alternatively, firstly, sleeving the locating cylinder outside the barrel, next, clamping the barrel flange into the mounting groove for fixing, and then connecting the connecting cylinder with the locating cylinder; with the method, the locating cylinder and the syringe can be fixed with respect to each other to form a sleeve outside the syringe, so as to provide an integral structure combining the syringe and the sleeve to facilitate the operator to use and operate; moreover, the syringe can lock the hub of the needle; thus, the injection will not be affected by inaccurate dosage caused by backward movement of the needle toward the syringe or deviation of the needle from the injection position under the effect of the tissues at the injection site in the injection process.

In the process that the operator applies thrust force on the push rod of the syringe to move the push rod in the syringe, since the hub of the needle is locked, the sleeve in the present disclosure prevents the tip of the needle from moving along with the movement of the push rod, so as to avoid excessive injuries of the target eye tissue or penetration through the target eye tissue owing to further extension of the needle into the target eye tissue.

In a preferred embodiment of the present application, the total length of the connecting cylinder and the locating cylinder is adjusted by means of the coupling between the connecting cylinder and the locating cylinder, or the total length of the connecting cylinder and the hub of the needle is adjusted by means of the couple between the connecting cylinder and the hub, thereby the relative length of the tip of the needle extending out of the injection end of the connecting cylinder is adjusted; an assembled integral structure is formed through the coupling between the connecting cylinder and the locating cylinder, and the integral structure enables adjusting the total length of the connecting cylinder and the locating cylinder by means of the couple between the connecting cylinder and the locating cylinder, thereby the exposed length of the tip of the needle can be adjusted.

In a preferred embodiment of the present application, after the barrel flange moves to the position corresponding to the mounting groove in the process of use, the barrel flange rotates along with the connecting cylinder, or the barrel flange is rotated and clamped into the mounting groove by means of manual rotation, and then the barrel is locked with the locating cylinder; through the rotary clamping of the syringe, the barrel can be coupled and fixed with the locating cylinder quickly and conveniently, and locking can be realized by the clamping.

In a fifth aspect, a method for using the aforesaid injection assembly is provided. The method comprises the following steps: selecting a syringe with a needle of a specific specification according to the requirements, and then fixing the syringe to the locating cylinder; and adjusting the length of the tip of the needle extending out of the connecting cylinder by adjusting the total length of the coupling between the connecting cylinder and the locating cylinder or the total length of the connecting cylinder and the hub of the needle. By selecting syringes with needles of different specifications, the needs of people of different ages can be met and the needs of different patients with different actual eye tissue thickness parameters can be met.

In a sixth aspect, a method for using the aforesaid injection assembly is provided. The method comprises the following steps: selecting a needle of a specific specification according to the requirements and placing the needle in the connecting cylinder; and forcing the needle to move toward the injection end of the injecting cylinder and adjusting the length of the tip of the needle extending out of the connecting cylinder by adjusting the total length of the couple between the connecting cylinder and the locating cylinder or the total length of the connecting cylinder and the hub of the needle. The operator can quickly select a needle of a specific specification according to the thickness of the patient's sclera, and can finely adjust the exposed length of the tip of the needle via the sleeve as required; thus, the operator can select the needle quickly and accomplish the assembling safely, the needs of persons of different ages and different patients with different actual eye tissue thickness parameters can be met, and the success ratio of injection can be improved.

In a preferred embodiment of the present application, when the connecting cylinder is coupled with the locating cylinder through threads from an end of the locating cylinder facing the tail part of the syringe push rod, the length of the tip of the needle extending out of the connecting cylinder can be adjusted by rotating the connecting cylinder in a reversed direction on a presumption that the direction of rotation of the connecting cylinder during the connection is positive. By changing the rotation mode of the connecting cylinder, the couple between the connecting cylinder and the locating cylinder is divided into two stages: one stage is rotary connection for coupling the connecting cylinder and the locating cylinder; the other stage is rotation in a reversed direction to realize the adjustment of the total length of the connecting cylinder and the locating cylinder, thereby adjust the exposed length of the tip of the needle.

In a preferred embodiment of the present application, the exposed length of the tip of the needle is 500 - 2,000 µm, preferably 700 - 1,350 µm, more preferably 700 -1,100 µm.

In a preferred embodiment of the present application, the blade length of the tip of the needle is smaller than or equal to 1,100 µm, preferably smaller than or equal to 900 µm, further preferably smaller than or equal to 700 µm, even more preferably smaller than or equal to 550 µm, optimally 250 - 550 µm.

In a preferred embodiment of the present application, the method further comprises: retaining the fingers by means of the limiting structure; holding the syringe sleeve in the hand, and making the clamping opening contact with and press the eye tissue at the site of injection, so that the eye tissue at the site of injection protrudes into the connecting cylinder; then forcing the tip of the needle to pierce through the eye tissue at the site of injection to a target eye tissue; and manipulating the push rod of the syringe to push the medicament into the target tissue. The fingers can be fixed on the outer side of the sleeve by means of the limiting structure; thus, the fingers of one hand can be fixed to the sleeve conveniently, and the eye tissue can be contacted and fixed by pressing through the clamping opening, so that the tip of the needle can penetrate to the target tissue; in that way, the operator can operate with one hand conveniently to inject the medicament into the eye tissue.

The syringe sleeve, the sleeve with a needle, the injection assembly and the method of use thereof provided in the embodiments of the present application can be widely applied to eye diseases and related diseases. The eye diseases include but are not limited to uveitis, glaucoma, diabetic macular edema, retinopathy, macular degeneration, retinoblastoma and genetic diseases.

In the present application, eye diseases can be treated by inserting a needle into the patient's sclera tissue to an effective length and delivering a medicament into the suprachoroidal space through the inserted needle with any device or method. In some embodiments, an effective amount of medicament administered to the suprachoroidal space provides a higher medicament therapeutic efficacy compared with the therapeutic efficacy achieved when the same amount of medicament is administered intravitreally, topically, intravenously, parenterally or orally. The medicament is delivered into the suprachoroidal space accurately with any device or method provided in the embodiments of the present application for subsequent local delivery to nearby posterior ocular tissues (e.g., retina and choroid) in need of treatment. In a time period after medicament administration, such as several hours, several days, several weeks or several months, the medicament can be released continuously in the eye tissue. In some embodiments, compared with oral, parenteral or intravitreal administration of the same dosage of medicament, any device or method provided in the embodiments of the present application can also improve the medicament bioavailability by local administration to eye tissues.

The syringe sleeve, the sleeve with a needle, the injection assembly and the method of use thereof provided in the embodiments of the present application are especially suitable for delivering medicaments to local posterior areas of eyes, such as retina and choroid tissues, macula and optic nerves in the posterior sections of eyes. The syringe sleeve, the sleeve with a needle, the injection assembly and the method of use thereof provided in the embodiments of the present application can also be used in gene-based therapy, in which a medical solution containing therapeutic gene fragments is delivered to the suprachoroidal space, and the therapeutic gene fragments are delivered to the target eye tissue in any one or more carriers selected from DNAs, RNAs or oligonucleotides.

As used herein, the term "medicament" refers to any preventive, therapeutic or diagnostic agent (e.g., contrast agent). The medicament may be selected from suitable proteins, peptides or fragments thereof, which can be naturally occurring, synthesized or recombinantly produced.

In some embodiments of the present application, the syringe contains a medicament solution of one or more pharmaceutically active substances, which may be selected from antibodies, antiviral agents, chemotherapeutic agents, analgesics, anesthetics, aptamers, antihistamines, anti-inflammatory agents and anti-tumor agents.

As used herein, the term "antibody" generally refers to any immunoconjugator, such as IgG, IgM, IgA, IgD and IgE. Antibodies can be monoclonal or polyclonal, and, in some embodiments, are humanized antibodies. The term "antibody" also refers to any antibody-like molecule that has an antigen-binding region and includes antibody fragments such as Fab", Fab, F(ab")₂, single-domain antibody (DAB), Fv, scFv (single-chain Fv) and engineered multivalent antibody fragments, such as bivalent antibody, trivalent antibody and multivalent antibody. Techniques for preparing and using antibodies and various structures and fragments based on antibodies are well known in the art.

In some embodiments of the present application, the syringe contains one or more genetic therapeutic medicaments that use recombinant adeno-associated viruses (rAAVs) as carriers, and the AAV is selected from AAV1, AAV2, AAV3, AAV4, AAV5, AAV6, AAV7, AAV8, AAV9, AAV10 or mutants thereof; preferably, the AAV is AAV8 or mutants thereof. The rAAV preferably contains medicaments expressed for treating eye diseases, such as anti-VEGF medicaments, complement factor inhibitors (complement component C3, complement factor B and complement factor D), plasma kallikrein inhibitors, PDGFR inhibitors, tyrosine kinase inhibitors, integrin inhibitors, angiopoietin-2 inhibitors and Tie-2 agonists.

In some embodiments of the present application, the syringe is a pre-filled syringe, which not only prevents the operator from hurting himself or causing inaccurate dosage during dispensing and decreases the probability of medicament misuse, but also effectively reduces residual medicament solution, so that the dosage of the medicament is more accurate, and the air can't contact with the medicament, thus avoiding cross-contamination.

In a seventh aspect, a kit is provided. The kit comprises: a vial containing a medicament solution; and an injection assembly; the injection assembly employs the aforesaid syringe sleeve or the aforesaid sleeve with a needle, the syringe is arranged in the connecting cylinder and the locating cylinder, and the barrel flange of the syringe is clamped in the mounting groove; the length of the needle of the syringe exposed from the connecting cylinder can be adjusted by means of the connection between the connecting cylinder and the locating cylinder or the connection between the connecting cylinder and the hub of the needle; the injection assembly can be combined with an existing syringe, thereby the length of the tip of the needle for injection into eye tissues can be adjusted conveniently to required length and accuracy to facilitate the operator to operate with one hand, and the use and operation are more convenient; moreover, when the connecting cylinder and the locating cylinder cooperate to adjust and limit the exposed length of the tip of the needle, the syringe in the locating cylinder can be quickly connected with the hub of the needle to form an integral structure, and the hub of the needle is locked.

In some embodiments of the present application, the medicament solution is a medicament solution that contains one or more pharmaceutically active substances, which may be selected from antibodies, antiviral agents, chemotherapeutic agents, analgesics, anesthetics, aptamers, antihistamines, anti-inflammatory agents and anti-tumor agents.

In some embodiments of the present application, the vial contains one or more genetic therapeutic medicaments that use recombinant adeno-associated viruses (rAAVs) as carriers, and the AAV is selected from AAV1, AAV2, AAV3, AAV4, AAV5, AAV6, AAV7, AAV8, AAV9, AAV10 or mutants thereof; preferably, the AAV is AAV8 or mutants thereof. The rAAV preferably contains medicaments expressed for treating eye diseases, such as anti-VEGF medicaments, complement factor inhibitors (complement component C3, complement factor B and complement factor D), plasma kallikrein inhibitors, PDGFR inhibitors, tyrosine kinase inhibitors, integrin inhibitors, angiopoietin-2 inhibitors and Tie-2 agonists.

In an eighth aspect, a kit is provided. The kit comprises: a pre-filled injection assembly; the pre-filled injection assembly employs the aforesaid syringe sleeve or the aforesaid sleeve with a needle and a pre-filled syringe, wherein the pre-filled syringe is arranged in a connecting cylinder and a locating cylinder, and a barrel flange of the pre-filled syringe is clamped in an mounting groove; and the length of the needle of the pre-filled syringe extending out of the connecting cylinder can be adjusted by means of the connection between the connecting cylinder and the locating cylinder or the connection between the connecting cylinder and the hub of the needle; the pre-filled injection assembly can be combined with an existing pre-filled syringe, thereby the length of the tip of the needle for injection into eye tissues can be adjusted conveniently to required length and accuracy to facilitate the operator to operate with one hand, and the use and operation are more convenient; moreover, when the connecting cylinder and the locating cylinder cooperate to adjust and limit the exposed length of the tip of the needle, the pre-filled syringe in the locating cylinder can be quickly connected with the hub of the needle to form an integral structure, and the hub of the needle is locked.

In some embodiments of the present application, the pre-filled syringe is pre-filled with a pharmaceutical solution of one or more pharmaceutically active substances, which may be selected from antibodies, antiviral agents, chemotherapeutic agents, analgesics, anesthetics, aptamers, antihistamines, anti-inflammatory agents and anti-tumor agents.

In some embodiments of the present application, the pre-filled syringe is pre-filled with one or more genetic therapeutic medicaments that use recombinant adeno-associated viruses (rAAVs) as carriers, and the AAV is selected from AAV1, AAV2, AAV3, AAV4, AAV5, AAV6, AAV7, AAV8, AAV9, AAV10 or mutants thereof; preferably, the AAV is AAV8 or mutants thereof. The rAAV preferably contains medicaments expressed for treating eye diseases, such as anti-VEGF medicaments, complement factor inhibitors (complement component C3, complement factor B and complement factor D), plasma kallikrein inhibitors, PDGFR inhibitors, tyrosine kinase inhibitors, integrin inhibitors, angiopoietin-2 inhibitors and Tie-2 agonists.

The medicament solution filling technique for the pre-filled syringe in the present disclosure is well known in the art.

Compared with the prior art, the embodiments of the present application achieve the following beneficial effects.
1. The syringe sleeve/sleeve with a needle provided in the embodiments of the present application utilizes the structure of an existing syringe, combines a syringe with a sleeve, and changes the operator's grip operation on the syringe into the operator's grip operation on the syringe sleeve, which is more convenient to use and operate; by positioning the syringe sleeve, the syringe can be positioned during injection into eye tissues, thereby a stable syringe position is provided, and backward movement of the syringe or deviation of the syringe from the injection position is avoided. Besides, the annular boss on the inner side of the injection end of the connecting cylinder is fitted with the raised ribs arranged on the periphery of the top end of the hub, so that the length of the tip of the needle extending out of the injection end of the connecting cylinder can be changed, thereby the tip of the needle can be positioned accurately, and the requirements of injection into eye tissues can be met; moreover, the coupling and adjustment can be completed quickly, and the syringe can lock the hub of the needle; it is unnecessary to carry out measurement and confirmation repeatedly during use, thereby time and labor are saved greatly; and the injection will not be affected by inaccurate dosage of the medicament resulted from backward movement of the syringe or deviation of the syringe from the injection position under the effect of the tissues at the site of injection during injection into eye tissues.
2. The syringe sleeve provided in the embodiments of the present application is coupled with the locating cylinder or the hub of the needle via the connecting end of the connecting cylinder, and the exposed length of the tip of the needle can be adjusted by adjusting the total length of the connecting cylinder and the locating cylinder or the total length of the connecting cylinder and the hub of the needle.
3. The syringe sleeve provided in the embodiments of the present application can facilitate the positioning of the fingers, and the operator can conveniently hold the syringe sleeve and fix the fingers with respect to the syringe sleeve, thereby the operation is more convenient.
4. In the syringe sleeve provided in the embodiments of the present application, by clamping the barrel flange to the locating cylinder, the syringe can be integrated with the locating cylinder, and the position of the connecting end of the connecting cylinder in the locating cylinder can be adjusted. The final position of backward movement of the connecting end is the position where the tip of the connecting end abuts against the barrel flange and the connecting end can't move backward further, thus, a function of preventing backward movement of the syringe sleeve is realized.
5. The injection assembly provided in the embodiments of the present application combines a sleeve with an existing syringe, thereby can meet different use requirements by selecting needles in lengths of different specifications; then, the exposed length of the tip of the needle can be adjusted by means of the coupling structure of the sleeve itself; when the sleeve is adjusted to a correct position, the exposed length of the tip of the needle reaches required length and accuracy and meets the requirements of eye injection; besides, the injection assembly is helpful for the operator to operate with one hand and is more convenient to use and operate.
6. The method for using the syringe sleeve provided in the embodiments of the present application can realize relative fixation between the locating cylinder and the syringe, form a sleeve outside the syringe, and provide an integral structure that combines the syringe with the sleeve, which is convenient for the operator to operate and use.
7. The method for using the injection assembly provided in the embodiments of the present application can meet the needs of persons of different ages and different patients with different actual eye tissue thickness parameters by selecting syringes with needles of different specifications; besides, the exposed length of the tip of the needle can be adjusted by means of the total length of the connecting cylinder and the locating cylinder or the total length of the connecting cylinder and the hub of the needle; thus, the needle can reach a predetermined position of eye tissue successfully, the injection effect is ensured, and the success ratio of injection is improved.
8. The injection assembly provided in the embodiments of the present application combines a sleeve with an existing syringe, the tip of the needle will not move backward toward the syringe or wobble during the penetration and lead to deviation of the needle from the injection position; the tip of the needle will not move when push force is applied to the push rod of the syringe to move the push rod in the syringe; thus, the tip of the needle is prevented from excessively extending in the target eye tissue, which may cause injuries of the target eye tissue or penetration through the target eye tissue.

### Brief Description of the Drawings

To explain the technical schemes in the embodiments of the present disclosure and in the prior art more clearly, the drawings to be used in the description of the embodiments and the prior art will be introduced below briefly. Obviously, the drawings used in the description below only illustrate some embodiments of the present disclosure, and those having ordinary skills in the art can work out other drawings based on these drawings without expending any creative labor.
Fig. 1 is a schematic exploded view of the syringe sleeve provided in an Embodiment 1 of the present application;
Fig. 2 is a schematic diagram of the needle used in the syringe sleeve in the Embodiment 1 of the present application;
Fig. 3 is a schematic sectional view of the injection end of the syringe sleeve in the Embodiment 1 of the present application, in which the tip of the needle is shown;
Figs. 4-6 are schematic diagrams of annular end faces in different shapes in different examples of the syringe sleeve in the Embodiment 1 of the present application;
Fig. 7 is a schematic diagram of the protrusion formed by the syringe sleeve and the eye tissue and the injection administration in the Embodiment 1 of the present application;
Fig. 8 is a schematic diagram of needles of different specifications used in the syringe sleeve in the Embodiment 1 of the present application;
Fig. 9 is a schematic connection diagram of the syringe sleeve in an Embodiment 2 of the present application;
Fig. 10 is a schematic diagram of the locating cylinder of the syringe sleeve in the Embodiment 2 of the present application;
Fig. 11 is a schematic diagram of the use of the syringe sleeve in the Embodiment 2 of the present application;
Fig. 12 is a schematic diagram of the syringe used in the embodiments of the present application;
Figs. 13-15 are schematic diagrams of limiting structures in different shapes used in different examples of the syringe sleeve in the Embodiment 2 of the present application;
Fig. 16 is a schematic cross-sectional view of the connecting cylinder of the syringe sleeve in the Embodiment 2 of the present application;
Fig. 17 is a partial sectional view of the syringe sleeve in the Embodiment 2 of the present application;
Fig. 18 is a combined schematic diagram of the syringe sleeve in an Embodiment 3 of the present application;
Fig. 19 is a schematic exploded view of the syringe sleeve in the Embodiment 3 of the present application;
Fig. 20 is a schematic connection diagram of the syringe sleeve in an Embodiment 4 of the present application;
Fig. 21 is a combined schematic diagram of the syringe sleeve in an Embodiment 5 of the present application;
Fig. 22 is a schematic exploded view of a syringe sleeve with a needle and a connecting cylinder in the Embodiment 6 of the present application;
Fig. 23 is a schematic connection diagram of the needle and the connecting cylinder in a first example of the syringe sleeve with a needle and a connecting cylinder in the Embodiment 6 of the present application;
Fig. 24 is a schematic connection diagram of a second example of the syringe sleeve with a needle and a connecting cylinder in the Embodiment 6 of the present application;
Fig. 25 is a schematic connection diagram of a third example of the syringe sleeve with a needle and a connecting cylinder in the Embodiment 6 of the present application;
Fig. 26 is an image of a fundus tissue section in a test example 1 of the present application;
Fig. 27 is an image of a fundus tissue section in a test example 4 of the present application;
Fig. 28 is an image of a fundus tissue section in a test example 5 of the present application;
Fig. 29 is an image of a fundus tissue section in a test example 6 of the present application;
Fig. 30 is an image of a fundus tissue section in a test example 7 of the present application;
Fig. 31 is an image of a fundus tissue section in a test example 8 of the present application;

Reference Numerals: 1 - connecting cylinder; 11 - injection end; 111 - annular boss; 112 - clamping opening; 113 - cavity; 114 - annular end face; 115 - contraction section; 12 - connecting end; 13 - positioning groove; 2 - locating cylinder; 21 - mounting groove; 22 - locking lug; 23 - flange mounting platform; 24 - limiting structure; 25 - limiting flange; 26 - arc-shaped protrusion; 3 - needle; 31 - raised rib; 32 - protrusion; 33 - hub; 34 - needle shaft; 4 - syringe; 41 - barrel flange; 5 - Luer connector.

### Detailed Description

The technical scheme of the present application will be described in detail in connection with test examples and specific embodiments. However, it may not be understood that the scope of the above-mentioned subject matter of the present application is limited to the following embodiments; instead, all techniques implemented on the basis of the content of the present disclosure belong to the scope of the present application.

The content of the applicant's prior application PCT/CN2022/107104 titled as Ophthalmic Injection Assembly, Injection Device and Use Method Therefor is incorporated into the present application as a part of the present application. The injection site, specification of needle, effective length of needle, blade length and blade penetration force are further explained as follows.

### 1. Injection site

The injection site described herein refers to any region of conjunctiva, such as superior nasal region, inferior nasal region, superior temporal region or inferior temporal region.

The injection site may be at any point in any region of conjunctiva between iris margin and corneal margin, such as superior nasal region, inferior nasal region, superior temporal region or inferior temporal region, at a distance of about 3-9 mm, about 4-8 mm, about 4-7 mm, about 6-8 mm, about 7-8 mm or about 4-5 mm from the corneal margin. The distance from the corneal margin may be about 3 mm, or about 4 mm, or about 5 mm, or about 6 mm, or about 7 mm, or about 8 mm.

2. Specification of needle: Existing commercial injection needles such as 28G, 30G, 31G, 32G, 33G and 34G may be selected, or customized injection needles produced through existing process of injection needles may be used.

Specification parameters of needle: The tip of the needle is usually configured to be sharp, beveled, or in other shapes that enable the needle to penetrate the ocular surface (e.g., sclera). The needle used may be of any suitable gauge, for example, about 25G, about 26G, about 27G, about 28G, about 29G, about 30G, about 31G, about 32G, about 33G, about 34G, about 35G, or about 36G. The wall of the needle may have any suitable thickness. For example, in addition to existing wall thickness (RW), the wall of the needle may be designed as a thin wall (TW), extra/ultra-thin wall (XTW/UTW), or extra-thin wall (XXTW). These names are well known to those skilled in the art. For example, the needle may be a fine gauge cannula or needle. In some variants, the needle may have a gauge between about 25G and about 36G. In other variants, the needle may have a gauge between about 27G and about 35G. In yet other variants, the needle may have a gauge between about 30G and about 33G.

### Effective length of needle:

The effective length of a needle is the length extending out of the clamping opening of the injection end of the connecting cylinder, and is about 1,400 µm or smaller, about 1,300 µm or smaller, about 1,200 µm or smaller, about 1,100 µm or smaller, about 1,000 µm or smaller, about 900 µm or smaller, about 800 µm or smaller, about 850 µm or smaller, about 700 µm or smaller, about 650 µm or smaller, about 500 µm or smaller, or about 450 µm or smaller. In some embodiments, the effective length of the needle may be about 700 µm. In other embodiments, the effective length of the needle may be about 750 µm, or about 800 µm, or about 850 µm, or about 900 µm, or about 950 µm, or about 1,000 µm, or about 1,100 µm, or about 1,350 µm.

### Blade length:

The blade length is the linear distance from the proximal inner edge of the needle wall at the liquid outlet of the tip of the needle to the tip edge of the outer wall of the liquid outlet of the tip of the needle, and is about 800 µm or smaller, about 700 µm or smaller, about 650 µm or smaller, about 600 µm or smaller, about 550 µm or smaller, about 500 µm or smaller, about 450 µm or smaller, about 400 µm or smaller, about 350 µm or smaller, about 300 µm or smaller, or about 250 µm or smaller. In some embodiments, the blade length may be about 550 µm; in other embodiments, the blade length may be about 700 µm, or about 650 µm, or about 600 µm, or about 500 µm, or about 450 µm, or about 300 µm, or about 250 µm.

### Blade penetration force:

The blade penetration force at the liquid outlet of the tip of the needle is about 0.7 N or smaller, about 0.65 N or smaller, about 0.5 N or smaller, about 0.4 N or smaller, or about 0.3 N or smaller, so as to reach to a desired position in the target tissue (e.g., suprachoroidal space and/or vitreous body) and form a medicament solution delivery channel. In some embodiments, the blade penetration force may be about 0.5 N; in other embodiments, the blade penetration force may be about 0.7 N, or about 0.65 N, or about 0.4 N, or about 0.3 N.

### needle shaft length:

The needle shaft length is the needle length extending out of the top end faces of the raised ribs of the hub, and is about 10 mm or smaller, about 9 mm or smaller, about 8 mm or smaller, about 7 mm or smaller, about 6 mm or smaller, about 5 mm or smaller, about 4 mm or smaller, or about 3 mm or smaller. In some embodiments, the needle shaft length may be about 6.5 mm. In other embodiments, the needle shaft length may be about 6.9 mm, or about 7.0 mm, or about 7.5 mm, or about 7.8 mm, or about 8.0 mm, or about 8.2 mm.

### 3. rAAV-anti-VEGF

As used herein, the term "rAAV-anti-VEGF" means that the rAAV contains a VEGF antagonist expressed for treating eye diseases, and the VEGF antagonist is preferably Aflibercept, Conbercept, Ranibizumab or Bevacizumab.

In addition, as used here, the term "syringe" has the definition commonly understood in the art, and may include a barrel and a push rod, wherein the barrel may be provided with a barrel flange at least for easing the holding by an operator. The "syringe" may be provided with a needle in advance, or the operator may choose a needle that is suitable for injection for the patient.

### Embodiment 1

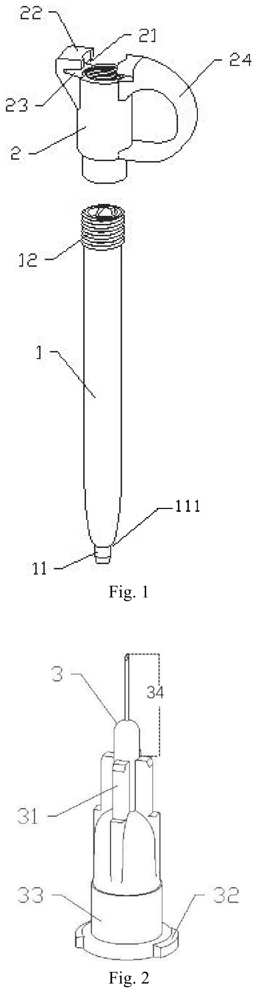

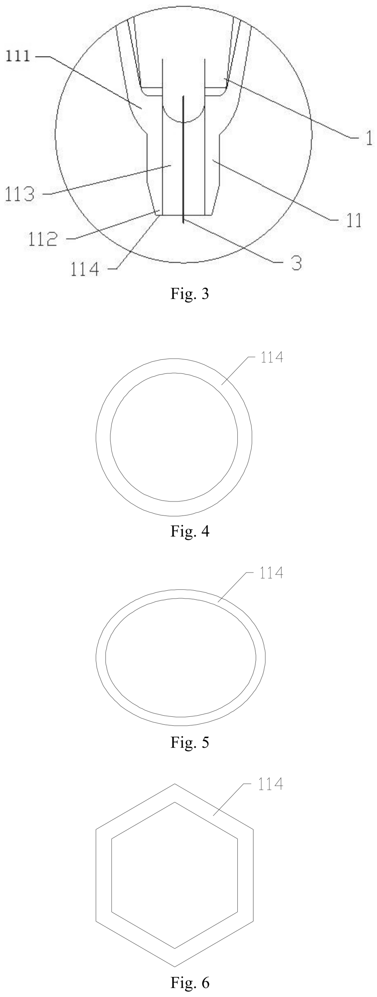

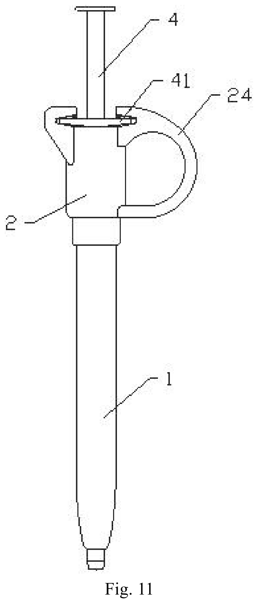

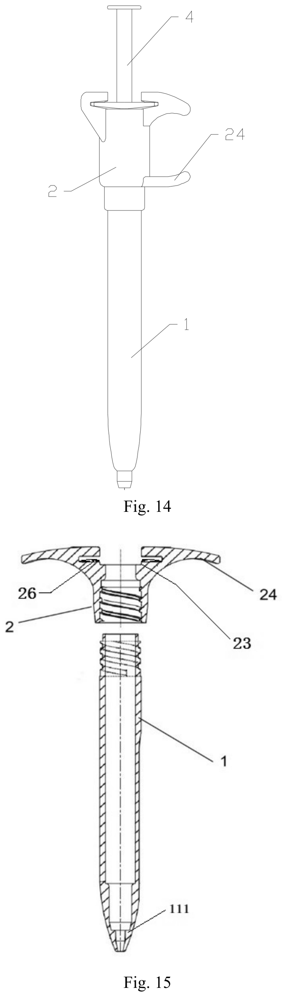

As shown in Figs. 1 and 3, in this embodiment, a syringe sleeve is provided. The syringe sleeve comprises a connecting cylinder 1 and a locating cylinder 2, both of which have a cylindrical structure, and the locating cylinder 2 is used to be sleeved outside a syringe 4 (Fig. 11); the two ends of the connecting cylinder 1 are an injection end 11 and a connecting end 12 respectively, and the injection end 11 is provided with an annular boss 111 and a clamping opening 112; as shown in Fig. 1, Fig. 3 and Figs. 15-17 to be described later, the annular boss 111 is a structure similar to a step around the inner side of the injection end 11, and the annular boss 111 is used for clamping the hub 33 of a needle 3, so as to limit the position of the needle 3. As shown in Fig. 2, since the hub 33 of the needle 3 is provided with raised ribs 31, and the hub 33 is clamped at the annular boss 111 by means of the raised ribs 31, the hub 33 can't move out of the injection end 11. Therefore, by means of the annular boss 111, the injection end 11 of the connecting cylinder 1 can limit the length of the needle 3 extending out of the connecting cylinder 1, thereby limit the exposed length of the tip of the needle 3 to a desired range to meet the requirements of injection for the tip of the needle 3. Thus, during the injection, the tip of the needle 3 can rightly reach the sclera in the eye tissues, and the needle is suitable for injection into a target eye tissue.

In this embodiment, the top end of the locating cylinder 2 is the end of the locating cylinder 2 facing the tail part of the push rod of the (mounted) syringe 4, and the bottom end of the locating cylinder 2 is the end of the locating cylinder 2 facing the connecting cylinder 1. The outer diameter of the connecting end 12 of the connecting cylinder 1 matches the inner diameter of the bottom end of the locating cylinder 2, and the connecting end 12 of the connecting cylinder 1 is inserted into and coupled with the locating cylinder 2 from the bottom end of the locating cylinder 2. The connecting end 12 of the connecting cylinder 1 is provided with external threads, and the inner wall of the bottom end of the locating cylinder 2 is provided with internal threads; through a threaded connection, the connecting end 12 can be connected to the locating cylinder 2 and the total length of the connecting cylinder 1 and the locating cylinder 2 can be adjusted by rotation. When the connecting end 12 of the connecting cylinder 1 is rotated to the end of the thread, the connecting cylinder 1 and the locating cylinder 2 are connected to the final state and can't be rotated any more. At that point, the tip of the needle 3 is exposed from the injection end 11 of the connecting cylinder 1 in a preset length.

Please see Fig. 2 further. In this embodiment, there are three raised ribs 31 on the periphery of the top end of the hub 33 of the needle 3, and the raised ribs are equally spaced on the periphery of the hub 33; the raised ribs 31 form ridges distributed longitudinally on the periphery of the hub 33 in the direction of the needle shaft 34, so that the outer diameter of the top end of the hub 33 is greater than the annular boss 111. The raised ribs 31 can be clamped to or abut against the annular boss 111, and the length of the needle 3 extending out of the injection end 11 can be limited by means of the annular boss 111. The raised ribs 31 facilitate the hub 33 to be clamped in the injection end 11 of the connecting cylinder 1, so that the length of the needle shaft 34 minus the distance from the annular boss 111 to the clamping opening 112 is the exposed length of the tip of the needle 3. During use, once the raised ribs 31 are clamped to the annular boss 111, the needle 3 reaches to the final position, and will no longer extend outward. Thus, by designing the positions of the needle shaft and the annular boss 111 precisely, it can be ensured that only the tip of the needle 3 in a preset length is exposed, so as to meet the requirements of injection into eye tissues. Moreover, when the connecting cylinder 1 and the locating cylinder 2 fixedly coupled via the barrel are rotated and tightened, the connecting cylinder 1 abuts against the barrel flange 41, and the raised ribs 31 of the needle 3 are clamped to the annular boss 111. Thus, by designing the positions of the barrel flange 41 and the annular boss 111 precisely, the exposed length of the tip of the needle 3 can be adjusted and the needle can be limited by means of the connecting cylinder 1 and the locating cylinder 2, and the hub 33 can be quickly coupled with the syringe in the locating cylinder 2 into an integral injection device, and the syringe 4 can lock the hub 33. From the above description, it can be seen that when the syringe sleeve provided in this embodiment is used, it is unnecessary to repeatedly measure and confirm the penetration length and position of the needle penetrating into the eye tissue before the injection, thereby time and labor can be saved, and the injected will not be affected by inaccurate dosage resulted from backward movement of the needle or deviation of the needle from the injection position under the effect of the eye tissues at the site of injection in the eye injection process.

In the process that the operator applies thrust force on the push rod of the syringe to move the push rod in the syringe, since the connecting cylinder 1 locks the hub 33 of the needle, the tip of the needle 3 will not move along with the movement of the push rod, thereby injuries of the target eye tissue or penetration through the target eye tissue owing to excessive extension of the needle in the target eye tissue can be avoided.

Please see Fig. 3. In this embodiment, the injection end 11 of the connecting cylinder 1 is the end facing the eye tissues, and is used to enable the tip of the needle 3 to extend out for an injection operation. The tip of the injection end 11 of the connecting cylinder 1, i.e., the end of the connecting cylinder 1 facing the eye tissue, is provided with a clamping opening 112, which is a circular opening for pressing the eye tissue at the site to be injected. The clamping opening 112 has an annular end face 114, which is the end face of the injection end 11 and is annular. The annular end face 114 includes an inner ring curve and an outer ring curve, with certain distance therebetween. Both the inner ring curve and the outer ring curve are smooth transition curves, and the space surrounded by the inner ring curve is the clamping opening 112. The shortest line segment that passes through the injection point and has two ends located in the curve of the inner ring, i.e., the minimum inner diameter of the annular end face of the clamping opening, is also referred to as the inner diameter of the clamping opening. The part between the inner ring curve and the outer ring curve is the wall thickness of the injection end 11. In this embodiment, the clamping opening 112 is a circular opening, and the annular end surface 114 is shown in Fig. 4. In other embodiments, an elliptical annular end face as shown in Fig. 5 or a polygonal annular end face as shown in Fig. 6 may be used. Clamping openings in different shapes, such as circular shape, elliptical shape, hexagonal shape, octagonal shape, square shape or irregular shape, can be used to press the eye tissues. The annular end face 114 is preferably a ring-shaped end face, which is not only more conducive to avoiding injuries of the conjunctiva but also more conducive to the restoration of the eye tissues during the injection.

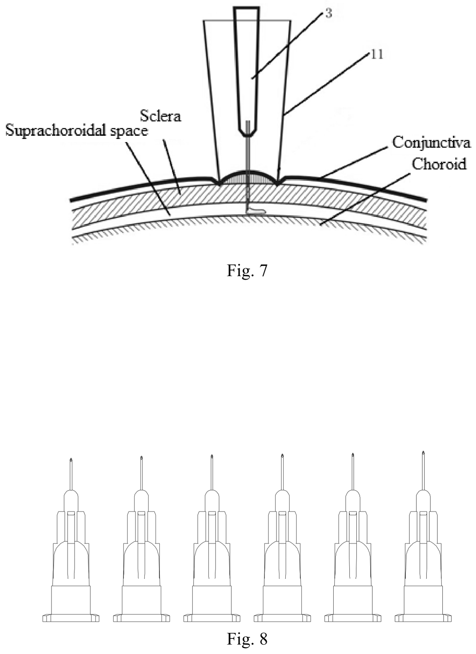

During use, as shown in Fig. 7, the circular edge of the clamping opening 112 contacts with and presses against the eye tissue, and the eye tissue at the position bulges into the injection end 11 at the clamping opening 112, and, at this time, the tip of the needle 3 penetrates into the target eye tissue at the site of injection. By clamping an appropriate amount of eye tissue with the clamping opening 112 and making the eye tissue bulges obviously in the clamping opening 112, the medicament solution can be delivered to the target site successfully, the success rate of injection can be improved, reflux can be effectively prevented, and diffusion of the medicament solution under the conjunctiva can be prevented.

As shown in Fig. 3, in this embodiment, the injection end 11 is further provided with a cavity 113, which is located in the injection end 11 near the clamping opening 112. The cavity 113 is cylindrical, and one end of the cavity 113 is closed by the hub 33, i.e., the hub 33 abuts against the cavity 113 and separates the cavity 113 from the connecting tube 1, and the other end of the cavity 113 is closed by the squeezing of the clamping opening 112 on the eye tissue. With the arrangement of the cavity 113, when reflux occurs, the medicament solution in the reflux will be contained in the closed cavity 113; when the clamping opening 112 is separated from the eye tissues, the medicament solution will be absorbed by the cavity 113 under a siphonage effect and will not leak out of the closed cavity 113 easily, thus, the medicament solution is prevented from dripping into the eye tissues.

In this embodiment, the injection end is a component made of a transparent material, such as polypropylene, so that the operator can observe the situation of reflux of the medicament solution conveniently and whether the injection is successful or not can be judged quickly. Alternatively, a viewing window made of a transparent material may be formed in the side wall of the injection end, and the effect of convenient observation can also be achieved by means of the viewing window; the transparent injection end or the transparent window can further be provided with scale lines, which are more conducive to accurately judging the situation of reflux of the medicament solution and observing the volume of the medicament solution reflux, thereby the injected amount of medicament solution can be calculated conveniently.

The needle 3 is available in a variety of specifications, and the syringe 4 can use the needles 3 of different specifications to meet the injection requirements. The needle shafts 34 of needles 3 of different specifications are in different lengths within a range of 5-10 mm. In this embodiment, the length of the needle shaft 34 of the needle 3 may be selected from 6.7 mm, 6.9 mm, 7.1 mm, 7.5 mm, 7.8 mm, 8.0 mm, 8.2 mm and 8.5 mm, etc. Needles 3 with needle shafts 34 in different lengths can be selected individually according to the different situations and needs of different patients.

### Embodiment 2

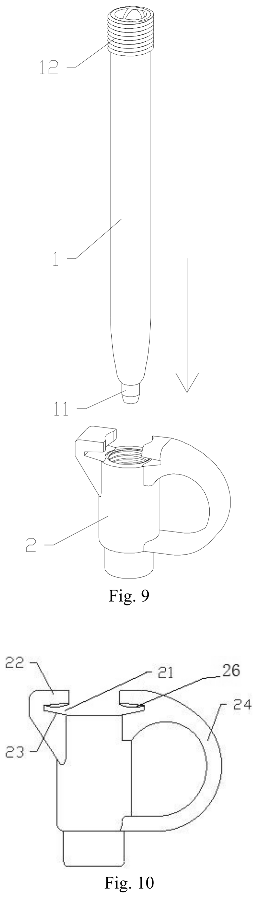

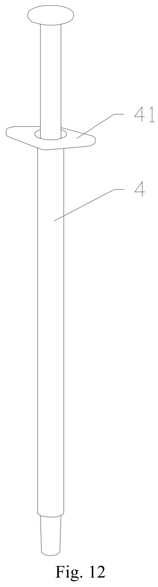

As shown in Figs. 9-10 and 12-17, in this embodiment, a syringe sleeve is provided. The structure of the syringe sleeve is essentially the same as the structure in the Embodiment 1, except the following differences: in this embodiment, the connecting cylinder 1 is provided therein with a positioning groove 13, and the locating cylinder 2 is provided with a mounting groove 21 for mounting the barrel flange41. The mounting groove 21 is a gap formed between the locking lug 22 and the flange mounting platform 23. The outer side of the locating cylinder 2 is further provided with a limiting structure 24. With the syringe sleeve, the barrel flange 41 can be fixed to the locating cylinder 2 by means of the structure of an existing syringe, so that the operator's grip operation on the syringe 4 can be changed into the operator's grip operation on the syringe sleeve, thereby the use and operation are more convenient. The total length of the connecting cylinder 1 and the locating cylinder 2 can be adjusted conveniently by means of the coupling between the connecting cylinder 1 and the locating cylinder 2, thus, the exposed length of the tip of the needle 3 can be adjusted by adjusting the relative positions of the connecting cylinder 1 and the locating cylinder 2, so that the tip of the needle 3 is positioned and the exposed length of the tip of the needle 3 can meet the requirements of injection into eye tissues.

As shown in Fig. 9, in this embodiment, the outer diameter of the connecting end 12 of the connecting cylinder 1 matches the inner diameter of the locating cylinder 2, and the connecting cylinder 1 is inserted into the locating cylinder 2 from the end of the locating cylinder 2 facing the tail part of the push rod of the syringe 4 (the upper end of the locating cylinder 2 in Fig. 9) for connection, which is to say, after the connecting cylinder 1 and the locating cylinder 2 enter a coaxial state, the injection end 11 of the connecting cylinder 1 is inserted into the locating cylinder 2 from the end of the locating cylinder 2 facing the tail part of the push rod of the syringe 4. After the insertion, the injection end 11 of the connecting cylinder 1 extends out from the other end of the locating cylinder 2 (the lower end of the locating cylinder 2 in Fig. 9). The connecting end 12 of the connecting cylinder 1 is provided with external threads, and the inner wall of the locating cylinder 2 is provided with internal threads. The end with the internal threads is spaced from the other end of the locating cylinder 2, thus, the external threads of the connecting cylinder 1 are always fitted with the internal threads of the locating cylinder 2 when the connecting cylinder 1 rotates, so as to keep the connection, and the connecting cylinder 1 will not be disengaged from the locating cylinder 2. The connecting end 12 is coupled to the locating cylinder 2 by means of rotation and the total length of the connecting cylinder 1 and the locating cylinder 2 is adjusted by means of rotation, and the relative length of the connecting cylinder 1 and the locating cylinder 2 can be adjusted by means of the threaded connection, thereby the total length can be adjusted. After the syringe 4 is mounted to the syringe sleeve, the barrel flange 41 abuts against the connecting end 12 and limits the rotary backward movement distance of the connecting end 12 in the locating cylinder 2. The syringe 4 can be integrated with the locating cylinder 2, and the position of the connecting end 12 of the connecting cylinder 1 in the locating cylinder 2 can be adjusted. The final backward movement position of the connecting end 12 is the position where the tip of the connecting end 12 abuts against the barrel flange 41. At this time, the connecting end 12 can't move backward anymore, thereby a backward movement prevention function of the connecting cylinder is realized.

As shown in Figs. 10-12, in this embodiment, the locating cylinder 2 is provided with a locking lug 22, a flange mounting platform 23 and a limiting structure 24; the top end of the locating cylinder 2 is the end of the locating cylinder 2 facing the tail part of the push rod of the syringe 4 after the syringe 4 is mounted, and the bottom end of the locating cylinder 2 is the end of the locating cylinder 2 facing the connecting cylinder 1 after the locating cylinder 2 is coupled with the connecting cylinder 1. The top end of the locating cylinder 2 is provided with the flange mounting platform 23, which is a platform for mounting the barrel flange 41 and is used for supporting one side of the barrel flange 41. The top end of the locating cylinder 2 is coupled with the locking lug 22 for clamping the other side of the barrel flange 41. The locking lug 22 comprises two structures symmetrically arranged at the top end of the locating cylinder 2. The locking lug 22 is in a shape of Arabic numeral 7, and the bottom end of the locking lug 22 is coupled with two sides of the flange mounting platform 23 of the locating cylinder 2. Two locking lugs 22 are arranged oppositely with a gap therebetween; the gap is used for the barrel flange 41 to pass therethrough in the vertical direction when the syringe 4 is rotated, so that the barrel flange 41 is clamped in a corresponding horizontal position of the mounting groove 21. There is a gap between the locking lug 22 and the flange mounting platform 23, and the gap is the mounting groove 21. After the barrel flange 41 being arranged to correspond to the mounting groove 21 at a horizontal position, the barrel flange 41 is rotated and clamped into the mounting groove 21 in the horizontal direction. The mounting groove 21 provides a space for clamping the barrel flange 41 on the locating cylinder 2, so that the syringe 4 can be fixed to the locating cylinder 2 conveniently. In this embodiment, the bottom of the locking lug 22 is provided with an arc-shaped protrusion 26 or the top of the flange mounting platform 23 is provided with the arc-shaped protrusion 26 for locking one side of the barrel flange 41. With the arrangement of the arc-shaped projection 26, the distance from the corresponding position of the arc-shaped projection 26 of the locking lug 22 to the flange mounting platform 23 is smaller than the thickness of the barrel flange 41, i.e., there is an interference fit between the barrel flange 41, the locking lug 22 and the flange mounting platform 23. With the arc-shaped projection 26, the barrel flange 41 is clamped and locked in the mounting groove 21, and the barrel flange 41 is in a locked state once it is clamped, so as to prevent the syringe 4 from being disengaged from the locating cylinder 2.

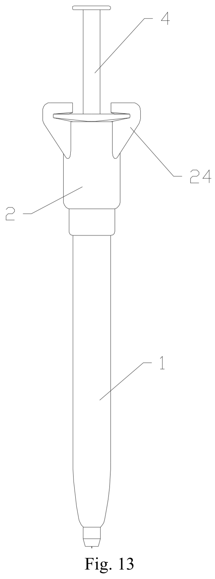

In this embodiment, the locating cylinder 2 is provided with a limiting structure 24, which is located at the outer side of the locating cylinder 2. As shown in Fig. 11, the limiting structure 24 may be a ring structure, which is located at one side of the locating cylinder 2 and used for clamping the fingers of the operator. With the ring structure, it is convenient to position the fingers. During use, the operator's index finger or other finger extends into and is fixed in the ring structure, so that the operator can hold the syringe sleeve conveniently and fix the finger with respect to the syringe sleeve to facilitate the operation. Alternatively, the limiting structure 24 may be a protrusion or recess structure, as shown in Figs. 13-15.

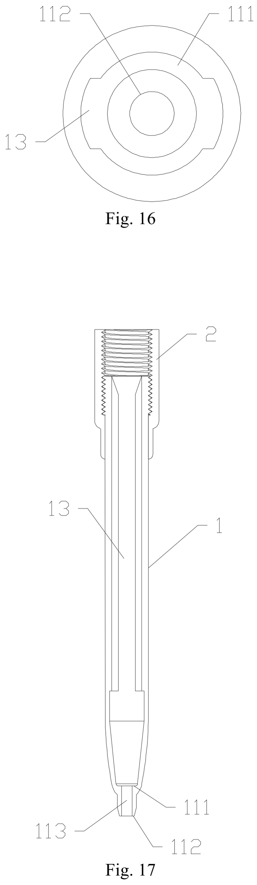

As shown in Figs. 16 and 17, in this embodiment, the connecting cylinder 1 is further provided with two positioning grooves 13 arranged at opposite positions, or may be provided with one or more positioning grooves 13. The positioning groove 13 is formed by the inner wall of the connecting cylinder 1 being recessed in the axial direction, thus, two positioning grooves 13 having the same length and arranged at opposite positions are formed in the inner wall of the connecting cylinder 1. The positioning groove 13 is used for clamping with the outer side of the hub 33 of the needle 3, or the outer side of the barrel may be configured in a shape matching the positioning groove 13. In this embodiment, protrusions 32 are provided on the outer sides of the hub 33, and the shape and size of the protrusions 32 match the cross section of the positioning groove 13; that is to say, protrusions 32 are provided on the two sides of the hub 33, and the two protrusions 32 limit the needle 3 in the positioning groove 13, so that the needle 3 can move axially in the connecting cylinder 1 along the positioning groove 13. When the protrusions 32 are moved across the positioning groove 13, the needle 3 can be released from the positioning groove 13 and rotate with respect to the connecting cylinder 1. In that way, when the syringe 4 is inserted, the syringe 4 always moves in one direction under the guidance of the needle 3 without wobble, thus, the needle 3 will not contact with the inner wall of the connecting cylinder 1 during the insertion of the syringe 4 and will be kept in a clean state.

### Embodiment 3

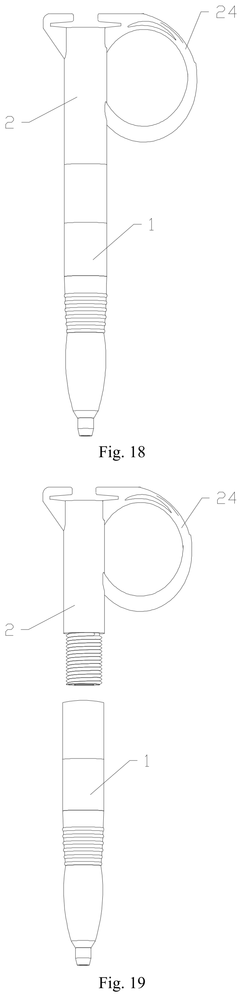

As shown in Figs. 18-19, in this embodiment, a syringe sleeve is provided. The structure of the syringe sleeve in this embodiment is essentially the same as the structure in the Embodiment 1, except the following differences: The connecting cylinder 1 is connected from the end of the locating cylinder 2 facing the needle 3, which is to say, the connecting end 12 of the connecting cylinder 1 abuts the bottom end of the locating cylinder 2 in opposite directions.

As shown in Fig. 19, in this embodiment, the injection end 11, the positioning groove 13 and the structure of the connecting cylinder 1 are the same as those in the Embodiment 1, but the length of the connecting cylinder 1 is shorter than that in the Embodiment 1. The bottom end of the locating cylinder 2 in this embodiment has an extension and the total length of the locating cylinder 2 is greater than that in the Embodiment 1. The connecting end 12 of the connecting cylinder 1 is provided with internal threads, and the extension at the bottom end of the locating cylinder 2 is provided with external threads. The inner diameter of the connecting end 12 of the connecting cylinder 1 matches the outer diameter of the extension of the locating cylinder 2, and the connecting cylinder 1 and the locating cylinder 2 can be connected through the threads. During the connection, when the connecting cylinder 1 is rotated to the top end of the extension with threads, the tip of the connecting end 12 of the connecting cylinder 1 rightly abuts against the top end of the extension of the locating cylinder 2; at this time, the total length of the connecting cylinder 1 and the locating cylinder 2 will not be decreased anymore, and the position is the final position of connection. In use, the syringe 4 is placed in the locating cylinder 2 first, and the barrel flange 41 is clamped into the mounting groove 21 of the locating cylinder 2 for fixation. At this time, the syringe 4 partially extends out of the locating cylinder 2; then, the connecting cylinder 1 is inserted from one end of the needle 3 of the syringe 4, so that the needle 3 of the syringe 4 is located at the injection end 11 of the connecting cylinder 1; next, the connecting cylinder 1 is rotated and connected with the locating cylinder 2. When the connecting cylinder 1 is rotated to the final position, the exposed length of the tip of the needle 3 is rightly equal to a preset length for injection into the eye tissues and meets the requirements of injection to eye tissues.

### Embodiment 4

In this embodiment, a syringe sleeve is provided. The structure of the syringe sleeve in this embodiment is essentially the same as the structure in the Embodiment 3, except the following differences: The connecting cylinder 1 is connected with the locating cylinder 2 through a snap-fit connection.

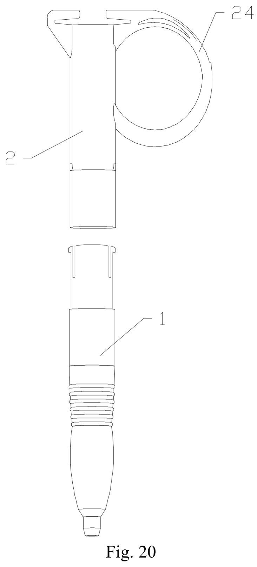

As shown in Fig. 20, in this embodiment, the structures of the injection end 11 of the connecting cylinder 1, the positioning groove 13 and the locating cylinder 2 are the same as those of in the Embodiment 3, but the connecting end 12 of the connecting cylinder 1 is provided with a fastener, and the inner side of the bottom end of the locating cylinder 2 is provided with a groove that matches the fastener. The outer diameter of the connecting end 12 of the connecting cylinder 1 is equal to the inner diameter of the bottom end of the locating cylinder 2, so that the connecting end 12 of the connecting cylinder 1 can be snap-fitted with the locating cylinder 2 by insertion to the locating cylinder 2. More specifically, the connecting end 12 of the connecting cylinder 1 is provided with fasteners on two sides, and the fasteners protrude outward on the two sides of the connecting end 12, and grooves in the inner wall of the bottom end of the locating cylinder 2 match the fasteners in shape and size; during use, the syringe 4 is placed into the locating cylinder 2 first, and the barrel flange 41 is clamped into the mounting groove 21 of the locating cylinder 2 and fixed there; at that point, the syringe 4 partially extends out of the locating cylinder 2. Then, the connecting cylinder 1 is sleeved on the syringe 4 from the end of the syringe 4 with the needle 3, so that the needle 3 is located at the injection end 11 of the connecting cylinder 1, and then the connecting cylinder 1 and the locating cylinder 2 are abut-jointed. When the connecting cylinder 1 is inserted into the locating cylinder 2 to a final position, the fastener snaps into the groove. At that point, the exposed length of the tip of the needle 3 meets the preset injection length for eye tissue injection and meets the requirements of eye tissue injection.

### Embodiment 5

In this embodiment, a syringe sleeve is provided. In this embodiment, only the connecting cylinder 1 is the same as the connecting cylinder in the Embodiment 2, the locating cylinder 2 only has the mounting groove 21 but has no limiting structure 24.

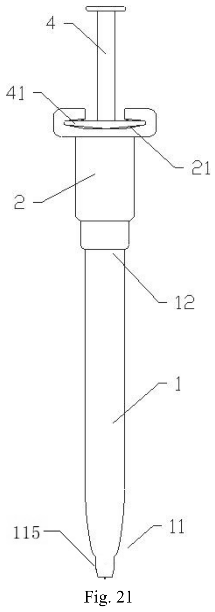

As shown in Fig. 21, in this embodiment, the two ends of the connecting cylinder 1 are an injection end 11 and a connecting end 12 respectively. The connecting end 12 of the connecting cylinder 1 is provided with external threads, the bottom end of the locating cylinder 2 is provided with internal threads, and the connecting end 12 of the connecting cylinder 1 is connected with the locating cylinder 2 through a threaded connection. The top end of the locating cylinder 2 is further provided with a mounting groove 21 for mounting a barrel flange 41. The mounting groove 21 is formed by the top end of the locating cylinder 2 in a recessed manner on the outside, so that the barrel flange 41 can be clamped in the mounting groove 21. The injection end 11 of the connecting cylinder 1 is provided with a contraction section 115, the cross-sectional area of the contraction section 115 gradually increases in the direction away from the clamping opening 112 of the injection end 11, wherein the internal opening of the contraction section 115 is smaller than the top end of the hub 33, so that the hub 33 of the needle 3 of the syringe 4 can be clamped and restricted from further moving, thereby a needle limiting function is achieved. The injection end 11 allows the needle shaft of the needle 3 to pass there-through, so that the tip of the needle 3 can be exposed at the injection end of 11 the connecting cylinder 1 after the connecting cylinder 1 is coupled with the locating cylinder 2. The syringe sleeve can change the original grip operation of the operator on the syringe 4 into a grip operation of the operator on the syringe sleeve, thereby the use and operation are more convenient. The total length of the connecting cylinder 1 and the locating cylinder 2 can be adjusted conveniently by means of the connection between the connecting cylinder 1 and the locating cylinder 2, thus, the exposed length of the tip of the needle 3 can be adjusted by adjusting the relative positions of the connecting cylinder 1 and the locating cylinder 2, so that the tip of the needle 3 is positioned and the exposed length of the tip of the needle 3 can meet the requirements of injection into eye tissues.

When the connecting cylinder 1 and the locating cylinder 2 adjust the exposed length of the tip of the needle 3 and limit the needle 3, the syringe 4 can lock the hub 33. It is unnecessary to carry out measurement and confirmation repeatedly during use, thereby time and labor can be saved greatly, and the injection will not be affected by inaccurate dosage caused by backward movement of the needle toward the syringe or deviation of the needle from the injection position under the effect of the tissues at the injection site in the injection process.

In the process that the operator applies thrust force on the push rod of the syringe to move the push rod in the syringe, since the syringe locks the hub 33 of the needle, the tip of the needle will not move along with the movement of the push rod, thereby injuries of the target eye tissue or penetration through the target eye tissue owing to excessive extension of the needle in the target eye tissue can be avoided.

### Embodiment 6

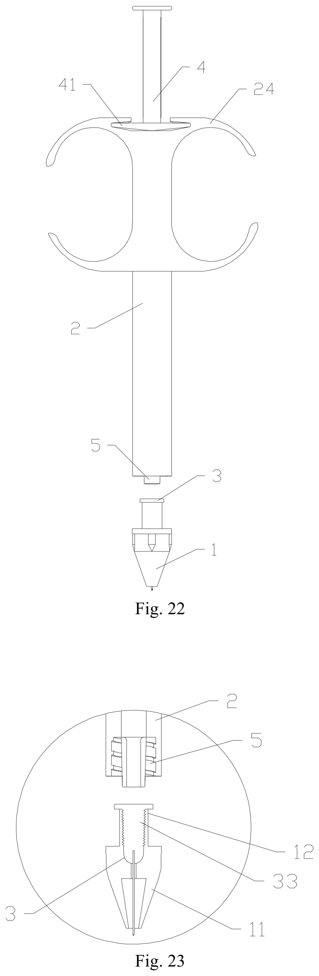

As shown in Figs. 22-25, in this embodiment, a (syringe) sleeve with a needle is provided. The syringe sleeve comprises the following components to be sleeved outside a syringe: a connecting cylinder 1 and locating cylinder 2. The connecting cylinder 1 and locating cylinder 2 in this embodiment employ the same structure as that in Embodiment 2, except the following differences: As shown in Fig. 22, the connecting cylinder 1 and the locating cylinder 2 are indirectly connected by means of a Luer connector 5, and the connecting cylinder 1 and the needle 3 are connected by means of threads, limiting structures 24 are arranged on two sides of the locating cylinder, and the connecting end 12 of the connecting cylinder 1 is coupled with the hub 33 of the needle 3. The total length of the connecting end 12 of the connecting cylinder 1 and the hub 33 can be adjusted. The total length from the connecting end 12 of the connecting cylinder 1 to the hub 33 can be changed for at least one time from the initial position and state of the connection to the final position and state of the connection, thus, the total length of the connecting cylinder 1 and the hub 33 can be changed.

As shown in Fig. 23, in this embodiment, the inner wall of the connecting end 12 of the connecting cylinder 1 is provided with a section of internal threads in the axial direction, while the outer side of the hub 33 is provided with external threads matching the connecting end 12. When the hub 33 and the connecting cylinder 1 are rotated and tightened in a forward direction, the tip of the needle 3 is exposed from the injection end 11 of the connecting cylinder 1, and the exposed length can rightly meet the requirements of injection into eye tissues. The coupling between the locating cylinder 2 and the barrel flange 41 is the same as that in the Embodiment 2, and the barrel is clamped into the mounting groove for fixation. After the locating cylinder 2 and the barrel are fixedly coupled, the syringe and the hub 33 are quickly coupled by means of a Luer connector 5 of the syringe 4 to form an integral injection device. The syringe sleeve is indirectly coupled with the connecting cylinder 1 through the needle 3, and the exposed length of the tip of the needle 3 is limited by the injection end 11, so that the tip of the needle 3 can be positioned, thereby the requirements of injection into eye tissues are met. Moreover, the connection and adjustment of needle 3 and the connecting cylinder 1 can be completed quickly, and it is unnecessary to make measurements and confirmations repeatedly during use, thus, time and labor are saved greatly.

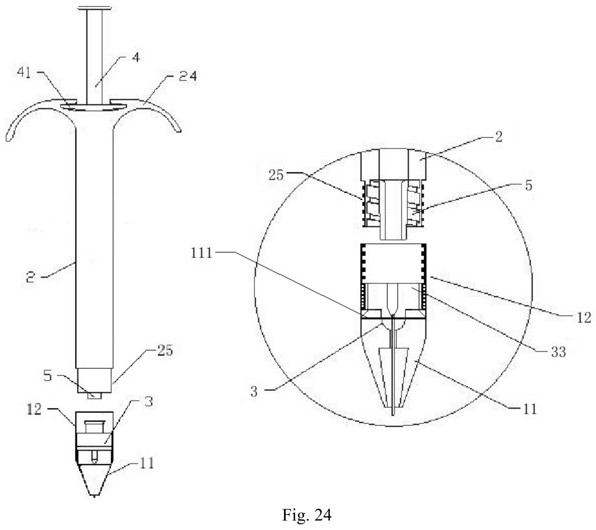

As shown in Fig. 24, the bottom end of the locating cylinder 2 is provided with a limiting flange 25. When the hub 33 and the connecting cylinder 1 are rotated and tightened, the connecting cylinder 1 moves toward the Luer connector 5 at the injection end of the syringe till it abuts against the limiting flange 25 of the locating cylinder 2, thus, backward movement of the connecting cylinder 1 can be prevented. At that point, the raised ribs 31 of the needle 3 are clamped to the annular boss 111. By precisely designing the positions of the limiting flange 25 and the annular boss 111, it is ensured that the hub 33 can be quickly connected with the Luer connector 5 to form an integral injection device, and the Luer connector 5 can lock the hub 33 while the exposed length of the tip of the needle 3 is limited by the injection end 11.

Thus, it is unnecessary to carry out measurement and confirmation repeatedly during use, thereby time and labor can be saved greatly, and the injection will not be affected by inaccurate dosage caused by backward movement of the needle toward the syringe or deviation of the needle from the injection position under the effect of the tissues at the injection site in the injection process.

In the process that the operator applies thrust force on the push rod of the syringe to move the push rod in the syringe, since the syringe locks the hub 33 of the needle, the tip of the needle will not move along with the movement of the push rod, thereby injuries of the target eye tissue or penetration through the target eye tissue owing to excessive extension of the needle in the target eye tissue can be avoided.

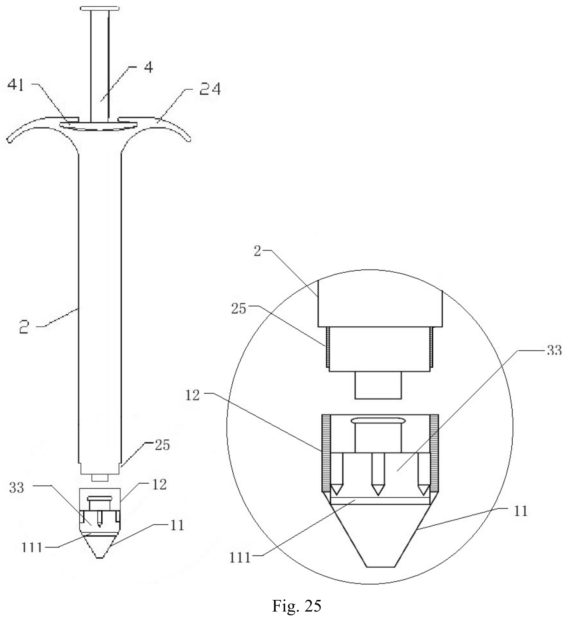

As shown in Fig. 25, in this embodiment, the outer wall of the locating cylinder between the limiting flange 25 and the bottom end of the locating cylinder 2 is provided with external threads that can be fitted with the internal threads of the connecting cylinder 1. After the hub 33 is put into the connecting cylinder 1 from the connecting end 12 of the connecting cylinder 1, the connecting end 12 is directly coupled with the bottom end of the locating cylinder 2 that is fixedly connected with the barrel. The connecting cylinder 1 and the locating cylinder 2 rotate relatively, so that the connecting cylinder 1 moves toward the bottom of the locating cylinder 2, and the tip of the needle 3 is exposed from the injection end 11 of the connecting cylinder 1, till the connecting end 12 abuts against the limiting flange 25 of the locating cylinder 2 and can't move anymore, and the raised ribs 31 of the needle 3 are clamped to the annular boss 111, thus, the exposed length of the tip of the needle 3 can meet the requirements of injection into eye tissues, and the syringe 4 locks the hub 33.

Thus, it is unnecessary to carry out measurement and confirmation repeatedly during use, thereby time and labor can be saved greatly, and the injection will not be affected by inaccurate dosage caused by backward movement of the needle toward the syringe or deviation of the needle from the injection position under the effect of the tissues at the injection site in the injection process. Moreover, the tip of the needle 3 can be prevented from being exposed out of the connecting cylinder 1 untimely, which may cause an increased risk of injuries of the operator or damage of the tip of the needle.

In the process that the operator applies thrust force on the push rod of the syringe to move the push rod in the syringe, since the syringe locks the hub of the needle, the tip of the needle will not move along with the movement of the push rod, thereby injuries of the target eye tissue or penetration through the target eye tissue owing to excessive extension of the needle in the target eye tissue can be avoided.

In this embodiment, by selecting needles 3 of different specifications for connection with the connecting end 12 of the connecting cylinder 1, the needs of people of different ages can be met and the needs of different patients with different actual eye tissue thickness parameters can be met.

In this embodiment, the outer wall of the locating cylinder between the limiting flange 25 and the bottom end of the locating cylinder 2 may be connected with the connecting cylinder 1 by means of threads, or may be connected by an existing connecting and fixing method such as plugging, snap-fitting or crimping.

### Embodiment 7

In this embodiment, an injection assembly is provided. The injection assembly may employ the syringe sleeve in the Embodiments 1-6. For example, as shown in Fig. 11, the injection assembly may comprise a syringe sleeve, a syringe 4 and a needle 3, and the injection assembly is an integrated structure in which the syringe 4 and the syringe sleeve are assembled. The syringe 4 is placed in the connecting cylinder 1 and the locating cylinder 2, and the barrel flange 41 of the syringe 4 is clamped in the mounting groove 21, thus, the syringe 4 is locked to the locating cylinder 2. By means of the connecting cylinder 1 and the locating cylinder 2 or the hub 33 of the needle 3, the length of the needle 3 of the syringe 4 extending out of the connecting cylinder 1 can be adjusted, and finally the exposed length of the needle 3 can be maintained at a preset length. The injection assembly can be combined with a syringe 4, thereby the length of the tip of the needle 3 can be adjusted conveniently to required length and accuracy to facilitate the operator to operate with one hand, and the use and operation are more convenient; moreover, when the connecting cylinder 1 and the locating cylinder 2 cooperate to adjust and limit the exposed length of the tip of the needle 3, the syringe 4 in the locating cylinder 2 can be quickly connected with the hub 33 of the needle to form an integral injection device, and the hub 33 is locked.

Thus, it is unnecessary to carry out measurement and confirmation repeatedly during use, thereby time and labor can be saved greatly, and the injection will not be affected by inaccurate dosage caused by backward movement of the needle toward the syringe or deviation of the needle from the injection position under the effect of the tissues at the injection site in the injection process.

In the process that the operator applies thrust force on the push rod of the syringe to move the push rod in the syringe, since the syringe locks the hub of the needle, the tip of the needle will not move along with the movement of the push rod, thereby injuries of the target eye tissue or penetration through the target eye tissue owing to excessive extension of the needle in the target eye tissue can be avoided.

### Embodiment 8

In this embodiment, a method for using a syringe sleeve is provided. The method can employ the syringe sleeve in the Embodiments 1 to 5. If the Embodiment 2 is employed, the method comprises:
A1. inserting the connecting cylinder 1 from the top end of the locating cylinder 2 first, and then inserting the syringe 4 into the connecting cylinder 1 and the locating cylinder 2 from the top end of the locating cylinder 2, so that the syringe 4 is completely inserted under the guidance of the positioning groove 13 of the connecting cylinder 1, and the needle 3 of the syringe 4 is located at the injection end 11 of the connecting cylinder 1; next, connecting the connecting cylinder 1 and the locating cylinder 2 through a threaded connection by rotation.
A2. rotating the connecting cylinder 1 further, so that the barrel flange 41 moves to a position corresponding to the mounting groove 21 along with the rotation of the connecting cylinder 1, then the barrel flange 41 is driven by the connecting cylinder 1 to be rotationally clamped into the mounting groove 21 for fixation or manually clamped into the mounting groove 21 for fixation; after the barrel flange 41 is clamped, the syringe 4 is locked to the locating cylinder 2; at that point, the tip of the needle 3 is located in the injection end 11 of the connecting cylinder 1 and is not exposed; the direction of rotation in the steps A1 and A2 is set as a forward direction.
A3. rotating the connecting cylinder 1 in a reverse direction, thereby the total length of the connecting cylinder 1 and the locating cylinder 2 is gradually decreased, and the connecting end 12 of the connecting cylinder 1 moves backward. When the connecting end 12 of the connecting cylinder 1 abuts against the surface of the barrel flange 41, the tip of the connecting end 12 of the connecting cylinder 1 is limited by the barrel flange 41, and the connecting cylinder 1 can't move backward further, so that the connecting cylinder 1 and the locating cylinder 2 reach a final position. At that point, the hub 33 of the needle 3 is clamped by means of the annular boss 111, and the tip of the needle 3 is exposed at the injection end 11 of the connecting cylinder 1 in a preset length, so that the hub 33 abuts against the syringe 4 and is locked by the syringe, and the needle 3 can't move backward further. By changing the rotation mode of the connecting cylinder 1, the connection between the connecting cylinder 1 and the locating cylinder 2 is divided into two stages: one stage is rotary connection for connecting the connecting cylinder 1 and the locating cylinder 2; the other stage is rotation in a reverse direction to adjust the total length of the connecting cylinder 1 and the locating cylinder 2, thereby adjust the exposed length of the tip of the needle 3, and enable the syringe 4 in the locating cylinder 2 to be connected quickly with the needle 3 to form an integral injection device and lock the hub 33.

If the syringe sleeve in the Embodiment 3 is employed, the method comprises: sleeving the locating cylinder 2 outside the cylinder of the syringe 4 first; then, clamping the barrel flange 41 into the mounting groove 21 for fixing; next, sleeving the connecting cylinder 1 outside the syringe 4, so that the needle 3 of the syringe 4 is located at the injection end 11 of the connecting cylinder 1; and finally, inserting the connecting cylinder 1 into the bottom end of the locating cylinder 2 and directly connecting them through snap-fitting. After the snap-fitted connection is formed, the exposed length of the tip of the needle 3 meets the preset length for injection into eye tissues.

### Embodiment 9

In this embodiment, a method for using a syringe sleeve is provided. The method employs the (syringe) sleeve with a needle in Embodiment 6, and the method comprises:
A1. sleeving the connecting end of the connecting cylinder 1 on the hub 33 of the needle 3 first; then, connecting the hub 33 via a Luer connector 5 or directly inserting the hub 33 into the end of a pre-filled syringe mounted in the locating cylinder 2 near the needle; next, connecting the connecting end of the connecting cylinder 1 with the hub 33 via a threaded connection by rotation. The connecting cylinder 1 rotates and moves on the hub 33 toward the needle 3, the total length of the injection end of the connecting cylinder 1 and the hub 33 starts to decrease gradually, and the connecting end 12 of the connecting cylinder 1 moves backward. When the connecting end 12 of the connecting cylinder 1 abuts against the root of the hub 33, the tip of the connecting end 12 of the connecting cylinder 1 is limited and clamped by the barrel flange 41, and the connecting cylinder 1 can't move backward further. At that point, the connecting cylinder 1 and the locating cylinder 2 reach a final position, while the hub 33 is clamped by the annular boss 111, and the tip of the needle 3 is exposed at the injection end 11 of the connecting cylinder 1, and the exposed length is the preset length.
A2. inserting the barrel into the locating cylinder 2 from the top end of the locating cylinder 2; rotating the locating cylinder 2 so that the barrel flange 41 moves to a position corresponding to the mounting groove 21 along with the rotation of the locating cylinder 2, and then the barrel flange 41 rotates and is clamped into the mounting groove 21 under the driving of the locating cylinder 2, or the barrel flange 41 is manually clamped into the mounting groove 21 for fixation. After the barrel flange 41 is clamped, the syringe 4 is locked to the locating cylinder 2.
A3. rotating the locating cylinder 2 with the barrel mounted therein in a reverse direction of the connecting cylinder 1 and connecting it with the outer wall of the hub 33; when the locating cylinder 2 with the barrel mounted therein moves to the Luer connector 5 of the syringe 4 in the reverse direction of the connecting cylinder 1, inserting the distal end of the barrel into the internal opening of the hub 33 till the connecting cylinder 1 abuts against the limiting flange 25 of the locating cylinder 2, so that the distal end of the barrel, i.e., the end where the needle is mounted on the syringe, locks the hub 33.

### Embodiment 10

In this embodiment, a method for using an injection assembly is provided. The injection assembly in the Embodiment 7 is used here; in an example where the syringe sleeve in the Embodiment 1 is used, the method comprises:
B1. mounting a medicament suction needle at the distal end of the barrel of the syringe 4, and completing a medicament suction operation;
B2. a needle 3 of a specific specification is selected and mounted at the needle mounting position of the syringe 4 according to the requirements;
B3. inserting the connecting cylinder 1 into the locating cylinder 2 to complete an initial connection, and then inserting the syringe 4 with the needle 3 into the connecting cylinder 1 and the locating cylinder 2;
B4. rotating the connecting cylinder 1 in the forward direction, so that the syringe 4 with the needle 3 is driven by the connecting cylinder 1 to rotate and the barrel flange 41 is clamped into a mounting groove 21, thus, the syringe 4 and the locating cylinder 2 are fixed together;
B5. rotating the connecting cylinder 1 in a reverse direction, so as to adjust the length of the tip of the needle 3 extending out of the connecting cylinder 1 by adjusting the total length of the connection between the connecting cylinder 1 and the locating cylinder 2, till the connecting cylinder 1 can't move backward further; at that point, the exposed length of the tip of the needle 3 is the preset length;
B6. extending a finger into the limiting structure 24 so that the finger is fixed with respect to the syringe sleeve, and holding the syringe sleeve in the same hand for operation;
B7. measuring the eye to be injected and confirming the injection position with a measuring instrument;
B8. holding the syringe sleeve, with the clamping opening 112 of the connecting cylinder 1 perpendicular to the surface of the eye tissue at the injection site;
B9. making the clamping opening 112 of the connecting cylinder 1 contact with the eye tissue at the injection site through the cooperation between the syringe 4 of the eye injection device and the syringe sleeve, and applying force to the eye tissue at the injection site, so that the conjunctival tissue clamped by the clamping opening 112 of the connecting cylinder 1 protrudes into the injection end 11 of the connecting cylinder 1, and the tip of the needle 3 is pressed into the target eye tissue at the injection site;
B10. pushing the push rod of the syringe 4 with the same hand or the other hand for injection, so that the medicament reaches the target tissue at the injection site.

In this method, needles 3 of different specifications can be selected to meet the needs of persons of different ages and patients having different actual eye tissue thickness parameters. By means of the limiting structure 24, the fingers can be fixed on the outer side of the sleeve, so that the fingers of a hand can be fixed with respect to the sleeve conveniently, and the eye tissue can be contacted and fixed by pressing through the clamping opening 112, thereby the tip of the needle 3 can penetrate to the target tissue, and the operator can inject a medicament into the eye tissue with one hand conveniently.

The method of use will be explained below in test examples. The contents of Test Examples 1 and 2 are also described in PCT/CN2022/107104.

### Test Example 1

The test was numbered as Test No. 1-1. The eye injection assembly and syringe having the structure described in the Embodiment 10 in PCT/CN2022/107104 were used to form an injection device. According to the normal thickness of the sclera tissue and choroid of New Zealand rabbits, a needle with effective length of 700 µm and blade length of 500±50 µm was used, and the clamping opening 112 of the sleeve was circular and had an inner diameter of 1.5 mm;

The effective length, i.e., the length of the tip of the needle 2 beyond the clamping opening 112, was 700 µm; that is to say, the distance from the tip of the needle 2 to the annular end surface was 700 µm; the needle 2 was essentially perpendicular to the annular end face;

Test method: After anesthesia with pentobarbital, procaine hydrochloride eye drops were used for ocular surface anesthesia, and injection in the suprachoroidal space was carried out with the injection assembly within 1-2 min after the eye drops were applied.
Tested animal: healthy New Zealand rabbits, 1.8-2.2 kg;
Injection frequency: single-point injection in the right eye;
Injected reagent: 0.2% ICG, 100 µl;
The method for injection into the suprachoroidal space was as follows:
   Step 1: measuring the distance with an ophthalmic caliper to determine an injection site;
   Step 2: sucking an injection reagent into the syringe, and applying force to one side of the clamping opening 112 via the syringe, so that the side of the clamping opening 112 adheres to the eye tissue at the injection site, and a fulcrum is formed on the eye surface at the injection site;
   Step 3: turning the injection device around the fulcrum to the other side of the clamping opening 112, so that the distal end of the needle 3 penetrates into the eye tissue at the injection site;
   Step 4: turning around the fulcrum further so that the other side of the clamping opening 112 covers the eye surface at the injection site closely, and the clamping opening 112 holds the eye tissue to form a bulge into the sleeve 3, and the distal end of the needle 3 is vertically pressed into the sclera to reach the target eye tissue at the injection site; and
   Step 5: injecting the medicament to the administration site.

In the injection process, the injection situation was recorded, including reflux, diffusion on eye surface, and subconjunctival residue, diffusion, bleeding or congestion. Then, the rabbit was killed, the eyeballs were dissected, and flat samples of fundus tissues were prepared, and the distribution of the ICG solution in the suprachoroidal space was recorded by taking photos.

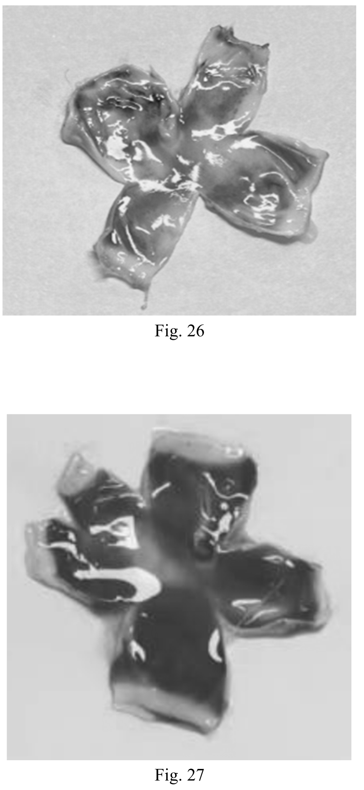

Test result: According to the observation result, there was no reflux or subconjunctival residue after the ICG injection. The result of fundus tissue section was shown in Fig. 26. All fundus tissues were transfected by ICG, and the dyed fundus tissues were in a light green color.

### Test Example 2

On the basis of the Test Example 1, the inner diameter of the clamping opening 112 was adjusted to investigate the effect of the inner diameter of the clamping opening 112 on the reflux of the medicament solution, while other conditions were kept unchanged. The inner diameters of the clamping openings 112 were 0.25 mm (Test No. 1-2), 0.5 mm (Test No. 1-3), 1.0 mm (Test No. 1-4), 2.0 mm (Test No. 1-5), 2.5 mm (Test No. 1-6), 3.0 mm (Test No. 1-7), 5.0 mm (Test No. 1-8), and 10.0 mm (Test No. 1-9) respectively.

The Test Example 1 and Test Example 2 are summarized as follows:

**Table 1 - Effect of Different Inner Diameters of Clamping Openings 112 on reflux**

| Test No. | Inner Diameter of Sleeve (mm) | Experimental Phenomena (Right Eye) | Experimental Phenomena (Tissue Section) |
|---|---|---|---|
| 1-1 | 1.5 | No reflux, no eye surface or subconjunctival diffusion. | All transfected by ICG, in light green color. |
| 1-2 | 0.25 | Obvious reflux, and obvious eye surface and subconjunctival diffusion | The reflux was serious, and the injection failed. The rabbit was not killed to make fundus slices. |
| 1-3 | 0.5 | Obvious reflux, and obvious eye surface and subconjunctival diffusion | The reflux was serious, and the injection failed. The rabbit was not killed to make fundus slices. |
| 1-4 | 1.0 | No reflux, no eye surface or subconjunctival diffusion. | The dyed area is about 3/4, in green color. |
| 1-5 | 2.0 | No obvious reflux, no eye surface diffusion, there was trace residue under the conjunctiva but no diffusion. | The dyed area is greater than 3/4, in dark green color. |
| 1-6 | 2.5 | No obvious reflux, no eye surface diffusion, there was trace residue under the conjunctiva but no diffusion. | The dyed area is about 2/3, in dark green color. |
| 1-7 | 3.0 | No obvious reflux, no eye surface diffusion, there were trace residue and small bulges under the conjunctiva but no diffusion. | The dyed area is about 3/5, in dark green color. |
| 1-8 | 5.0 | No reflux, no eye surface diffusion, and no residue under the conjunctiva. | The fundus tissue was not stained, but the vitreous body was stained. |
| 1-9 | 10.0 | No reflux, no eye surface diffusion, and no residue under the conjunctiva. | The fundus tissue was not stained, but the vitreous body was stained. |

The experimental result showed that when the inner diameter was 1-3 mm, there was no obvious reflux or diffusion on the eye surface or under the conjunctiva, and the tissue section result showed that the stained area was between 3/5 and 3/4, and successful injection was realized.

### Test Example 3

Syringe: BD, disposable sterile syringe with a needle, batch no.: 300841.
Needle: 30G
Syringe sleeve: the sleeve in the Embodiment 1
Injected reagent and volume: 100 µl
Test steps:
   1. 10 syringes were used, 100 µl purified water was sucked into each syringe respectively; 10±2N injection fixing force was used to simulate eye administration. The syringe was weighed before and after the administration, and the dosage and its relative standard deviation (RSD) were calculated.
   2. The syringe in the step 1 was used, and 100 µl purified water was sucked into each syringe again; each syringe was loaded into the sleeve described in the Embodiment 1 respectively; after the assembly, the injection fixing force described in step 1 was used to simulate eye administration; the syringe and the fixer were weighed before and after the administration, and the dosage and its relative standard deviation (RSD) were calculated to investigate the effect of the syringe sleeve provided in the embodiments of the present application on the accuracy of dosage administration.

Test result: With existing dosage and its relative standard deviation (as shown in Table 2) before and after the administration with the syringe sleeve in the embodiments of the present application, it is proved that the syringe sleeve in the present disclosure can significantly improve the accuracy of dosage.

**Table 2 - Dosage and Its Relative Standard Deviation (RSD) before and after Administration with the Syringe Sleeve**

| Group | Syringe No. | Before Administration (g) | After Administration (g) | Dosage (g) | RSD (%) |
|---|---|---|---|---|---|
| | Syringe 1 | 3.3011 | 3.2131 | 0.0880 | |
| | Syringe 2 | 3.3490 | 3.2370 | 0.1120 | |
| | Syringe 3 | 3.3316 | 3.2278 | 0.1038 | |
| | Syringe 4 | 3.3565 | 3.2556 | 0.1009 | |
| Without syringe sleeve | Syringe 5 | 3.3201 | 3.2142 | 0.1059 | 8.89 |
| | Syringe 6 | 3.3602 | 3.2782 | 0.0820 | |
| | Syringe 7 | 3.3333 | 3.2276 | 0.1057 | |
| | Syringe 8 | 3.3314 | 3.2286 | 0.1028 | |
| | Syringe 9 | 3.3739 | 3.2694 | 0.1045 | |
| | Syringe 10 | 3.3362 | 3.2338 | 0.1024 | |
| | Syringe 1 | 10.8107 | 10.7172 | 0.0935 | |
| | Syringe 2 | 10.8596 | 10.7592 | 0.1004 | |
| | Syringe 3 | 10.8316 | 10.7397 | 0.0919 | |
| | Syringe 4 | 10.8386 | 10.7382 | 0.1004 | |
| With syringe sleeve | Syringe 5 | 10.8228 | 10.7231 | 0.0997 | 3.73 |
| | Syringe 6 | 10.8458 | 10.7481 | 0.0977 | |
| | Syringe 7 | 10.8256 | 10.7219 | 0.1037 | |
| | Syringe 8 | 10.8240 | 10.7218 | 0.1022 | |
| | Syringe 9 | 10.8502 | 10.7531 | 0.0971 | |
| | Syringe 10 | 10.8566 | 10.7591 | 0.0975 | |

### Test Example 4

The syringe sleeve and syringe in the Embodiment 1 were used to form an injection assembly, in which the inner diameter of the clamping opening 112 of the connecting cylinder 1 was 1.5 mm and the blade length was 450±50 µm.

Test method: After anesthesia with pentobarbital, procaine hydrochloride eye drops were used for ocular surface anesthesia, and injection in the suprachoroidal space was carried out with the self-made injection device within 1-2 min after the eye drops were applied.
Tested animal: healthy New Zealand rabbits, 1.8-2.2 kg;
Injection frequency: single-point injection in the right eye;
Injected reagent: 0.2% ICG, 100 µl;
Injection method:
   The method for injection into the suprachoroidal space is as follows:
   Step 1: mounting a medicament suction needle into the distal end of the barrel of a syringe 4, completing the medicament suction operation, and removing the medicament suction needle and replacing it with a needle 3;
   Step 2: inserting the connecting cylinder 1 from the top end of the locating cylinder 2 first, and then inserting the syringe 4 into the connecting cylinder 1 and the locating cylinder 2 from the top end of the locating cylinder 2, so that the syringe 4 is completely inserted under the guidance of the positioning groove 13 of the connecting cylinder 1, and the needle 3 of the syringe 4 is located at the injection end 11 of the connecting cylinder 1; next, connecting the connecting cylinder 1 and the locating cylinder 2 through a threaded connection by rotation;
   Step 2: rotating the connecting cylinder 1 further, so that the barrel flange 41 moves to a position corresponding to the mounting groove 21 along with the rotation of the connecting cylinder 1, then the barrel flange 41 is driven by the connecting cylinder 1 to be rotationally clamped into the mounting groove 21 for fixation or manually clamped into the mounting groove 21 for fixation; after the barrel flange 41 is clamped, the syringe 4 is locked to the locating cylinder 2; at that point, the tip of the needle 3 is located in the injection end 11 of the connecting cylinder 1 and is not exposed; the direction of rotation here is set as a forward direction;
   Step 3: rotating the connecting cylinder 1 in a reverse direction, thereby the total length of the connecting cylinder 1 and the locating cylinder 2 is gradually decreased, and the connecting end 12 of the connecting cylinder 1 moves backward. When the connecting end 12 of the connecting cylinder 1 abuts against the surface of the barrel flange 41, the tip of the connecting end 12 of the connecting cylinder 1 is limited and clamped by the barrel flange 41, and the connecting cylinder 1 can't move backward further, the syringe 4 locks the hub 33 of the needle 1, so that the connecting cylinder 1 and the locating cylinder 2 reach a final position. At that point, the hub 33 of the needle 3 is clamped by the annular boss 111, and the tip of the needle 3 is exposed from the injection end 11 of the connecting cylinder 1 in a preset length. By changing the rotation mode of the connecting cylinder 1, the connection between the connecting cylinder 1 and the locating cylinder 2 is divided into two stages: one stage is rotary connection for realizing the connection between the connecting cylinder 1 and the locating cylinder 2; the other state is rotation in a reverse direction to adjust the total length of the connecting cylinder 1 and the locating cylinder 2, thereby adjust the exposed length of the tip of the needle 3; the exposed length of the tip of the needle 3 is adjusted to 0.9 mm;
   Step 4: measuring the eye to be injected and confirming the injection position with a measuring instrument;
   Step 5: holding the syringe sleeve, with the clamping opening 112 of the connecting cylinder 1 perpendicular to the surface of the eye tissue at the injection site;
   Step 6: making the clamping opening 112 of the connecting cylinder 1 contact with the eye tissue at the injection site through the cooperation between the syringe 4 of the eye injection device and the syringe sleeve, and applying force to the eye tissue at the injection site, so that the conjunctival tissue clamped by the clamping opening 112 of the connecting cylinder 1 protrudes into the injection end 11 of the connecting cylinder 1 to form a protrusion 32, and the tip of the needle 3 is pressed into the target eye tissue at the injection site;
   Step 7: pushing the push rod of the syringe 4 with the same hand or the other hand for injection, so that the medicament reaches the target tissue at the injection site.

In the injection process, the injection situation was recorded, including whether the needle 3 moved toward syringe 4, the reflux of the medicament solution, diffusion on eye surface, and subconjunctival residue, diffusion, bleeding or congestion; whether the tip of the needle deviated from the injection site and whether any scratch was produced on the eye surface during the injection were observed; then, the rabbit was killed, the eyeballs were dissected, and flat samples of fundus tissues were prepared, and the distribution of the ICG solution in the suprachoroidal space was recorded by taking photos.

Test result: The observation result showed that the needle 3 didn't move toward the syringe 4, there was no reflux or no diffusion on the eye surface, there was no residue, bleeding or congestion under the conjunctiva, there was no deviation from the injection site, and there was no scratch on the eye surface near the injection site. The result of fundus tissue section is shown in Fig. 27. Almost all the fundus tissues were transfected by ICG, and the stained fundus tissues were in a dark green color. The above experimental result proved that the injection was successful.

### Test Example 5

The differences from the Test Example 4 lie in: the inner diameter of the clamping opening 112 of the connecting cylinder 1 was 1.0 mm, the blade length was 450±50 µm, and the exposed length of the tip of the needle 3 was 0.7 mm.

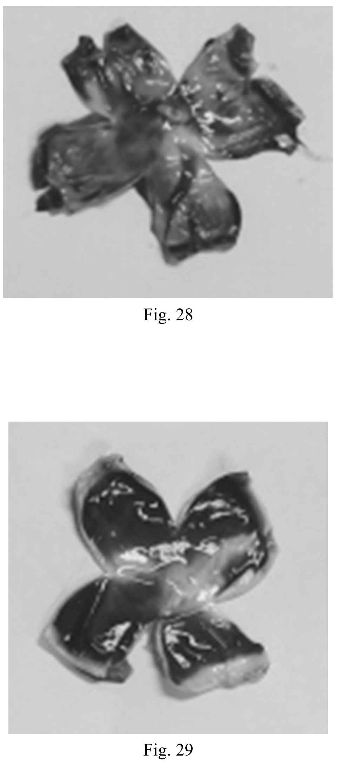

Test result: The observation result showed that the needle 3 didn't move toward the syringe 4, there was no reflux or no diffusion on the eye surface, there was no residue, bleeding or congestion under the conjunctiva, there was no deviation from the injection site, and there was no scratch on the eye surface near the injection site. The result of fundus tissue section is shown in Fig. 28. Almost all the fundus tissues were transfected by ICG, and the stained fundus tissues were in a green color. The above experimental result proved that the injection was successful.

### Test Example 6

The differences from the Test Example 4 lie in: the inner diameter of the clamping opening 112 of the connecting cylinder 1 was 3.0 mm, the blade length was 450±50 µm, and the exposed length of the tip of the needle 3 was 0.7 mm.

Test result: The observation result showed that the needle 3 didn't move toward the syringe 4, there was no reflux or no diffusion on the eye surface, there was no residue, bleeding or congestion under the conjunctiva, there was no deviation from the injection site, and there was no scratch on the eye surface near the injection site. The result of fundus tissue section is shown in Fig. 29. The area of the fundus tissues transfected by ICG exceeded 4/5, and the stained fundus tissues were in a dark green color. The above experimental result proved that the injection was successful.

### Test Example 7

The differences from the Test Example 4 lie in: the syringe sleeve, syringe and injection device in the Embodiment 2 were employed, and the blade length was 500±50 µm;

Injection method: A needle 3 with stem length of 6.8 mm was selected and mounted on the syringe 4 according to the requirements, and the exposed length of the tip of the needle 3 was 0.8 mm. A finger of the operator was placed on the limiting structure 24, and the finger was fixed with respect to the syringe sleeve, while the syringe sleeve was held in the same hand for operation.

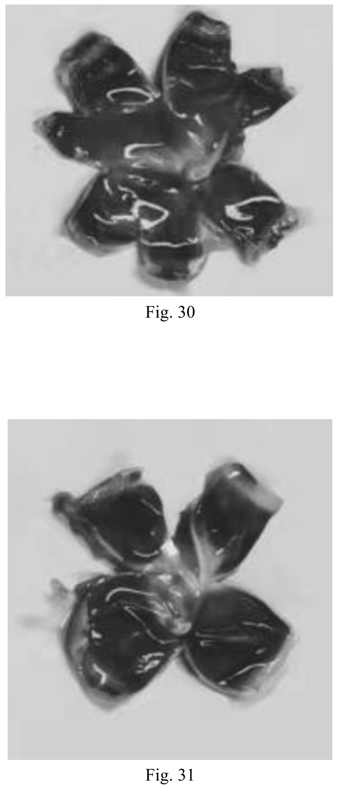

Test result: The observation result showed that the needle 3 didn't move toward the syringe 4, there was no medicament reflux, no diffusion on the eye surface and no residue, bleeding or congestion under the conjunctiva in the injection. The result of fundus tissue section is shown in Fig. 30. Almost all the fundus tissues were transfected by ICG, and the dyed fundus tissues were in a dark green color, and there was no deviation from the injection site, and there was no scratch on the eye surface near the injection site. The above experimental result proved that the injection was successful.

### Test Example 8

The differences from the Test Example 4 lie in: the syringe sleeve, syringe and injection device in the Embodiment 6 were employed, and the blade length was 350±50 µm;
Injection method:
Step 1: inserting the cylinder of the syringe 4 into the locating cylinder 2 from the top end of the locating cylinder 2, and rotating the cylinder of the syringe 4 to move the barrel flange 41 into the mounting groove 21, thus completing the fixing of the syringe 4 and the locating cylinder 2 with respect to each other;
Step 2: mounting a medicant suction needle at the distal end of the barrel of the syringe 4, and completing a medicant suction operation;
Step 3: selecting a needle 3 with stem length of 6.7 mm, and placing the needle 3 in the connecting cylinder 1;
Step 4: removing the medicament suction needle, and inserting the connecting cylinder 1 with a needle 3 into the distal end of the barrel;
Step 5: rotating the connecting cylinder 1 in the forward direction, and moving the connecting cylinder 1 with the needle 3 toward the distal end of the barrel, so that the tip of the needle 3 moves to the injection end of the connecting cylinder 1; adjusting the length of the tip of the needle 3 extending out of the connecting cylinder 1 by adjusting the total length of the connection between the connecting cylinder 1 and the locating cylinder 2; finally the connecting cylinder 1 abuts against the barrel and can't move backward, and the exposed length of the tip of the needle 3 is the present length of 0.7 mm;
Step 6: measuring the eye to be injected and confirming the injection position with a measuring instrument;
Step 7: holding the syringe sleeve, with the clamping opening 112 of the connecting cylinder 1 perpendicular to the surface of the eye tissue at the injection site;
Step 8: making the clamping opening 112 of the connecting cylinder 1 contact with the eye tissue at the injection site through the cooperation between the syringe 4 of the eye injection device and the syringe sleeve, and applying force to the eye tissue at the injection site, so that the conjunctival tissue clamped by the clamping opening 112 of the connecting cylinder 1 protrudes into the injection end 11 of the connecting cylinder 1 to form a protrusion 32, and the tip of the needle 3 is pressed into the target eye tissue at the injection site;
Step 9: pushing the push rod of the syringe 4 with the same hand or the other hand for injection, so that the medicament reaches the target tissue at the injection site.

Test result: The observation result showed that the needle 3 didn't move toward the syringe 4, there was no reflux or no diffusion on the eye surface, there was no residue, bleeding or congestion under the conjunctiva, there was no deviation from the injection site, and there was no scratch on the eye surface near the injection site. The result of fundus tissue section is shown in Fig. 31. Almost all the fundus tissues were transfected by ICG, and the stained fundus tissues were in a dark green color. The above experimental result proved that the injection was successful.

### Test Example 9

The injection device and the injection method described in the Embodiment 4 were used.
Tested animal: one rhesus monkey
Injection frequency: a single injection, OS(Left eye); the day of administration to the animal was defined as the first day of the test;
Injected reagent: rAAV-anti-VEGF
Dosage and concentration: dosage: 1.0x10¹² vg / eye, concentration: 1.0x10¹³ vg/ml.
Administration volume: 100 µl / eye.

Administration method: After the rhesus monkey was anesthetized, the eye to be injected was disinfected with povidone iodine solution; under an operating microscope, the eyelid was opened with an eyelid opener and the site of operation was exposed; the injection device described in the Test Example 4 was used, a needle 3 with stem length of 6.7 mm was selected, and the exposed length of the tip of the needle 3 was adjusted to 0.7 mm; from the back of the vitreous flat part of the vitreous body, the needle penetrated through the sclera above the superior temporal region of the eyeball to complete the injection and administration into the suprachoroidal space. After the operation, Ofloxacin eye ointment was applied in droplets to the two eyes to prevent corneal wetting and protect against infection.

In the injection process, the injection situation was recorded, including whether the needle 3 moved toward syringe 4, the reflux of the medicament solution, diffusion on eye surface, and subconjunctival residue, diffusion, bleeding or congestion; whether the tip of the needle deviated from the injection site and whether any scratch was produced on the eye surface during the injection were observed;
13 weeks after the administration, the rhesus monkey was sacrificed, and important ocular tissues such as aqueous humor, vitreous body, iris/ciliary body, retina, choroid and sclera were collected, and qPCR test was carried out to investigate the concentration distribution of the vector genome DNA in the ocular tissues.

Wherein:
Quantitative limit: concentration of vector genome DNA= 2.0 x 10⁴ vg/ml;
Tissue sample detection limit: 2.5 x 10⁴ vg/g tissue;
Detection limit of aqueous humor and vitreous body samples: 1.25 x 10³ vg/ml aqueous humor and vitreous body sample;
Test result:
   The injection observation result showed that the needle 3 didn't move toward the syringe 4, there was no reflux or no diffusion on the eye surface, there was no residue, bleeding or congestion under the conjunctiva, there was no deviation from the injection site, and there was no scratch on the eye surface near the injection site.

Through the detection of the concentration distribution of the target gene in the eye tissues (as shown in Table 3), it is known that the target gene is mainly distributed in sclera and choroid, and the above test is in line with the characteristics of injection in the suprachoroidal space.

**Table 3 - Concentration Distribution of Target Gene in Main Eye Tissues**

| Acquisition Time | Eye | Iris/Ciliary Body (vg/g) | Retina (vg/g) | Choroid (vg/g) | Sclera (vg/g) | Aqueous Humor (vg/ml) | Vitreous Body (vg/ml) |
|---|---|---|---|---|---|---|---|
| The 13th week | OS | 6.56E+08 | 1.93E+07 | 1.52E+10 | 8.58E+10 | BLD | BLQ |

| | | | | | | | |
|---|---|---|---|---|---|---|---|
| Note: BLQ means "below the quantitative limit" and BLD means "below the detection limit". | | | | | | | |

Through the observation of the injection situation and the concentration distribution of the target gene in the eye tissues, it is shown that the injection into the suprachoroidal space in this test was successful.

While the present application is described above in some preferred embodiments, the present application is not limited to those embodiments. Any modification, equivalent substitution, or improvement, etc. made under the spirit and principle of the present application may be deemed as falling in the scope of protection of the present application. The arrangement of parts can be adjusted adaptively for the specific parts arranged in specific orientations or positions as shown in the above schematic diagrams and/or embodiments. Similarly, the orders of specific methods and/or steps described herein may be adjusted. Although the embodiments have been particularly shown and described, it may be understood that various modifications in form and details may be made.

Although the device and method described herein are described and illustrated herein as being used to deliver a medicament in the suprachoroidal space, in other embodiments, the device and method described herein can be applied to deliver any suitable therapeutic substance to any part of the eye, such as cornea, conjunctiva, retinal region or vitreous body. In other embodiments, any of the device and method described herein can be used to deliver any suitable therapeutic substance to any desired target eye tissue.

## Claims

1. A syringe sleeve, comprising the following components for being sleeved on a syringe:
a locating cylinder for being fixed to a barrel flange, and
a connecting cylinder comprising a connecting end and an injection end, wherein the connecting end is used for connecting to the locating cylinder, and the injection end is used for limiting the part of the tip of a needle extending out of the connecting cylinder.

2. The syringe sleeve of claim 1, wherein when the connecting cylinder and the locating cylinder are coupled with each other, the total length of the connecting cylinder and the locating cylinder can be changed by means of the coupling.

3. The syringe sleeve of claim 1 or 2, wherein an annular boss is provided on an inner side of the injection end for clamping a hub of the needle and limiting the needle.

4. The syringe sleeve of claim 3, wherein multiple raised ribs are provided on the periphery of a top end of the hub of the needle for being clamped on the annular boss and limiting the length of the needle extending out of the injection end by the annular boss.

5. The syringe sleeve of claim 3, wherein when the length of the tip of the needle extending out of the connecting cylinder is adjusted via the injection end, the annular boss clamps the hub of the needle.

6. The syringe sleeve of claim 1 or 2, wherein the injection end is provided with a contraction section for clamping the hub of the needle and limiting the needle.

7. The syringe sleeve of any of claims 1 to 6, wherein the tip of the injection end is provided with a clamping opening for pressing the eye tissues at the site of injection, and the clamping opening is provided with an annular end face, which has a minimum inner diameter of 1 mm to 3 mm.

8. The syringe sleeve of any of claims 1 to 7, wherein the connecting end is coupled with the locating cylinder through a threaded connection or snap-fitted connection.

9. The syringe sleeve of claim 8, wherein the connecting end is configured to be coupled with the locating cylinder from an end of the locating cylinder facing the needle, or the connecting end is configured to be inserted into the locating cylinder and coupled with the locating cylinder from an end of the locating cylinder facing the tail end of a syringe push rod.

10. The syringe sleeve of claim 9, wherein the connecting end is provided with external threads, the locating cylinder is provided with internal threads, and the connecting end can be connected to the locating cylinder in a rotating way and adjust the total length of the connecting cylinder and the locating cylinder in a rotating manner.

11. The syringe sleeve of claim 9, wherein in the case that the connecting cylinder is configured to be inserted into and connected with the locating cylinder from an end of the locating cylinder facing the tail end of the syringe push rod, after the syringe is mounted, the barrel flange abuts against the connecting end and limits the backward rotation distance of the connecting end in the locating cylinder.

12. The syringe sleeve of any of claims 1 to 11, wherein the inner wall of the connecting cylinder is provided with a positioning groove in the axial direction for mutual clamping with an outer side of the barrel or an outer side of the hub of the needle, so that the syringe can move in the axial direction of the connecting cylinder under the guidance of the positioning groove.

13. The syringe sleeve of claim 12, wherein a protrusion is provided on the outer side of the hub of the needle, and the shape and size of the protrusion match the cross section of the positioning groove.

14. The syringe sleeve of any of claims 1 to 13, wherein the locating cylinder is provided with a mounting groove for mounting the barrel flange, so that the barrel flange is clamped into the mounting groove in a rotating manner.

15. The syringe sleeve of claim 14, wherein the locating cylinder is provided with a flange mounting platform for supporting one side of the barrel flange and a locking lug for clamping the other side of the barrel flange, and a gap between the locking lug and the flange mounting platform forms the mounting groove.

16. The syringe sleeve of claim 15, wherein an arc-shaped protrusion structure is provided at the top of the flange mounting platform or the bottom of the locking lug for locking to one side of the barrel flange.

17. The syringe sleeve of any of claims 1 to 16, wherein the part of the tip of the needle extending out of the connecting cylinder is limited by the injection end, so that the syringe locks the hub of the needle.

18. The syringe sleeve of any of claims 1 to 17, wherein a limiting structure for fixing the fingers of an operator is provided on the outer side of the locating cylinder, and the limiting structure is a protrusion, notch or ring structure.

19. A sleeve with a needle, comprising the following components for being sleeved on a syringe:
a locating cylinder for being fixed to a barrel flange, and
a connecting cylinder comprising a connecting end and an injection end, wherein the connecting end is configured for coupling with the locating cylinder;
the injection end is coupled with the needle therein by means of a hub of the needle, and limits the part of the tip of the needle extending out of the injection end.

20. The sleeve with a needle of claim 19, wherein when the injection end and the hub are coupled with each other, the total length of the injection end and the hub can be changed by means of the coupling.

21. The sleeve with a needle of claim 19 or 20, wherein an annular boss is provided on an inner side of the injection end of the connecting cylinder for clamping the hub of the needle and limiting the needle.

22. The sleeve with a needle of claim 19 or 20, wherein the injection end of the connecting cylinder is provided with a contraction section for clamping the hub of the needle and limiting the needle.

23. The sleeve with a needle of any of claims 19 to 22, wherein the syringe locks the hub when the injection end limits the part of the tip of the needle extending out of the injection end.

24. The sleeve with a needle of any of claims 19 to 23, wherein a Luer connector is provided between an end of the locating cylinder facing the connecting cylinder and the hub of the needle, so that the hub of the needle can be coupled quickly with the syringe in the locating cylinder to form an integral structure.

25. The sleeve with a needle of any of claims 19 to 23, wherein the connecting end of the connecting cylinder is coupled with the bottom end of the locating cylinder fixedly coupled with the barrel after it is coupled with the hub of the needle, so that the hub is coupled quickly with the syringe in the locating cylinder to form an integral structure.

26. An injection assembly, comprising the syringe sleeve of any of claims 1 to 18 or the sleeve with a needle of any of claims 19 to 25, and a syringe arranged in the connecting cylinder and the locating cylinder, wherein a barrel flange of the syringe is clamped in a mounting groove of the locating cylinder, and the length of the needle of the syringe extending out of the connecting cylinder is adjusted by means of a coupling between the connecting cylinder and the locating cylinder or a coupling between the connecting cylinder and the hub of the needle.

27. The injection assembly of claim 26, wherein when the length of the needle of the syringe extending out of the connecting cylinder is adjusted by means of the coupling between the connecting cylinder and the locating cylinder or the coupling between the connecting cylinder and the hub of the needle, the syringe in the locating cylinder can be quickly coupled with the needle to form an integral structure, and the hub of the needle is locked.

28. The injection assembly of claim 26 or 27, wherein the syringe is a pre-filled syringe.

29. A method for using the injection assembly of any of claims 26 to 28, **characterized in that** the syringe locks the needle by making the locating cylinder coupled with the connecting cylinder.

30. A method for using the injection assembly of any of claims 26 to 28, **characterized in that** the tip of the needle doesn't move with the movement of the push rod in the syringe by making the locating cylinder coupled with the connecting cylinder.

31. A method for treating eye diseases, comprising delivering a pharmaceutically active substance to the eye tissues of a subject in need of treatment with the injection assembly of any of claims 26 to 28, wherein the pharmaceutically active substance is selected from at least one of antibodies, antiviral agents, chemotherapeutic agents, analgesics, anesthetics, aptamers, antihistamines, anti-inflammatory agents, antineoplastic agents and gene therapeutic medicaments that employ a recombinant adeno-associated virus as a carrier.
